# EUROPEAN PATENT SPECIFICATION

(11) **EP 2 313 782 B1**
(45) Date of publication and mention of the grant of the patent: **29.08.2012**
(21) Application number: 09772535.2
(22) Date of filing: 03.07.2009
(51) Int. Cl.: G01N 33/68, G01N 33/50, G01N 33/92, G01N 33/98

(54) **PROTEOMIC FINGERPRINT FOR THE DIAGNOSIS OF NON-ALCOHOLIC STEATOHEPATITIS (NASH) AND/OR STEATOSIS**
PROTEOM-FINGERABDRUCK ZUR DIAGNOSE NICHTALKOHOLISCHER STEATOHEPATITIS (NASH) UND/ODER STEATOSE
EMPREINTE PROTÉOMIQUE POUR LE DIAGNOSTIC DE LA STÉATOHÉPATITE NON ALCOOLIQUE (NASH) ET/OU DE LA STÉATOSE

(30) Priority: 03.07.2008 EP 08380196
(43) Date of publication of application: 27.04.2011
(73) Proprietor: One Way Liver Genomics, S.L., 48160 Derio - Vizcaya (ES)
(72) Inventor: MATO DE LA PAZ, José María, E-48160 Derio (ES); GALÁN COUSILLAS, Asier, E-20180- OIARTZUN (ES); CASTRO ESPIDO, Azucena, E-48160 DERIO (ES); ELORTZA BASTERRIKA, Félix, E-48160 DERIO (ES)
(74) Representative: ABG Patentes, S.L.
(86) International application number: PCT/EP2009/058401
(87) International publication number: WO 2010/000835

(56) References cited:
- WO-A-03/038444
- US-A1- 2007 037 221
- US-B1- 6 631 330
- POYNARD THIERRY ET AL: "Diagnostic value of biochemical markers (NashTest) for the prediction of non alcoholo steato hepatitis in patients with non-alcoholic fatty liver disease" BMC GASTROENTEROLOGY, BIOMED CENTRAL LTD., LONDON, GB, vol. 6, no. 1, 10 November 2006 (2006-11-10), page 34, XP021022807 ISSN: 1471-230X
- SANTOS-GONZALEZ ET AL: "Modifications of plasma proteome in long-lived rats fed on a coenzyme Q10-supplemented diet" EXPERIMENTAL GERONTOLOGY, ELSEVIER SCIENCE, OXFORD, GB, vol. 42, no. 8, 18 July 2007 (2007-07-18), pages 798-806, XP022156547 ISSN: 0531-5565
- NICE ET AL: "Use of multidimensional separation protocols for the purification of trace components in complex biological samples for proteomics analysis" JOURNAL OF CHROMATOGRAPHY, ELSEVIER SCIENCE PUBLISHERS B.V. AMSTERDAM, NL, vol. 1168, no. 1-2, 2 October 2007 (2007-10-02), pages 190-210, XP022282439 ISSN: 0021-9673
- LINKE ET AL: "Rat plasma proteomics: Effects of abundant protein depletion on proteomic analysis" JOURNAL OF CHROMATOGRAPHY B: BIOMEDICAL SCIENCES & APPLICATIONS, ELSEVIER, AMSTERDAM, NL, vol. 849, no. 1-2, 7 April 2007 (2007-04-07), pages 273-281, XP022024446 ISSN: 1570-0232

## Description

### FIELD OF THE INVENTION

The invention relates to the field of pharmacoproteomics and, more in particular, to a proteomic signature formed by a collection of biomarkers for non-alcoholic steatohepatitis (NASH) and/or steatosis. Said biomarkers can be used in methods for diagnosing NASH and/or steatosis, more specifically for early diagnosis of NASH and/or steatosis, as well as in methods for determining the predisposition of a subject to develop NASH and/or steatosis, and for monitoring the effect of the therapy administered to said subject with said conditions.

### BACKGROUND OF THE INVENTION

Western countries account for a population of more than 200 million people suffering from obesity thus considered in risk of developing a non-alcoholic fatty liver disease (NAFLD). NAFLD is a clinic-pathologic term including disorders ranging from the simple accumulation of triglycerides into the hepatocytes (hepatic steatosis) to the hepatic steatosis with inflammation (Non-Alcoholic Steatohepatitis or NASH).

Fatty liver, also named hepatic steatosis, is defined as an excessive accumulation of fat in hepatocytes. Fatty liver disease involves the accumulation of triglycerides in hepatocytes, necrosis of hepatocytes, inflammation, small hepatic veins obliteration and often fibrosis with sometimes progression to cirrhosis, hepatocellular cancer and liver-related death (El-Serag H B, et al. Gastroenterology 2004;126:460-468, Dam-Larsen S, et al. Gut 2004;53:750-5).

Worldwide prevalence of hepatic steatosis is very high, associated with several factors such as alcohol, diabetes, overweight, hyperlipidemia, insulin resistance, hepatitis C genotype 3, abetalipoproteinemia and some drugs (Bellentani S, et al. Ann. Intern. Med. 2000;132:112-7; Levitsky J, Mailliard M E. Semin. Liver Dis. 2004;24:233-47). However, there is no standard recommendation for the diagnosis of hepatic steatosis. The usual recommendation is to measure GGT and ALT and to perform liver biopsy for the grading and staging (Bellentani S, et al. Ann. Intern. Med. 2000; 132:112-7; Levitsky J, Mailliard M E. Semin. Liver Dis. 2004;24:233-47; Bravo A A, et al. N. Engl. J. Med. 2001:344;495-500). As liver biopsy is still an invasive and costly procedure, with a potential sampling error, it is advantageous to have a test that would give a good predictive value of the level of hepatic steatosis in the subject.

The international patent application WO2006/082500 discloses a method for diagnosis of hepatic steatosis in a subject, comprising studying 5 biochemical markers by measuring the values of their concentration in the serum or plasma of said subject. Said markers are: ApoA1 (apolipoprotein A1), alpha.2-macroglobulin, ALT (alanine aminotransferase), GGT (gammaglutamyl transpeptidase) and triglycerides.

Non-alcoholic steatohepatitis (NASH) is a progressive disease of the liver of unknown etiology characterized histologically by fatty acid accumulation, hepatocyte damage and inflammation resembling alcoholic hepatitis. NASH is a critical stage in the process that spans from hepatic steatosis to cirrhosis and liver failure. A careful history of a lack of significant alcohol intake is essential to establish this diagnostic. NASH is one of the most common causes of elevated aminotransferases in subjects referred for evaluation to hepatologists. Obesity and type-2 diabetes are associated to NASH. Since the prevalence of these diseases is increasing, the prevalence of NASH is also expected to increase and therefore, this disease has become an emerging public issue in the United States as well as in other countries.

NASH is thought to arise from the interaction of many different genes and life style factors. Initial evaluation of subjects suspected of NASH when present, are fatigue and right upper abdominal discomfort. Liver biopsy is indicated in symptomatic subjects with serum aminotransferase elevations of more than 6 months duration. The histological features of NASH, which are mostly indistinguishable from those of alcoholic hepatitis, include macrovesicular fatty infiltration, hepatocellular necrosis and ballooning degeneration, alcoholic hyaline and inflammatory reaction.

As it is true for other complex diseases, the genetic factors contributing to the development of NASH may be more readily identified by combining studies in subjects with NASH and in animal models of the disease. One of these models is the *MAT1A* knockout (MAT1A-KO) mouse. At about 3 months of age, MAT1A-KO livers have normal histology but they are more sensitive to develop severe steatosis. These mice spontaneously develop NASH and hepatocellular carcinoma at about 8 and 15 months of age, respectively [Lu SC, 2001, PNAS USA 98, 5560-5565]. *MAT1A* gene encodes for methionine adenosyltransferase I and III, the main enzymes responsible of S-adenosylmethionine (SAMe) synthesis in the liver. Earlier studies concluded that subjects with liver cirrhosis and alcoholic hepatitis are deficient in SAMe synthesis; and that treatment with SAMe improves survival in subjects with alcoholic liver cirrhosis.

The international patent application WO2008/021192 discloses a method for diagnosing, assessing the severity and/or assessing the progression or regression of a liver disorder in a subject, such as hepatic steatosis or NASH. The method comprises determining an amount of one or more lipid metabolites in samples from a body fluid of the subject and correlating the amount(s) of the one or more lipid metabolites with the present of the liver disorder.

US 6631330 discloses marker sets, methods and kits to diagnose and monitor liver fibrosis and/or the presence of liver necroinflammatory lesions in a patient. Said method involves the study of at least 4 biochemical markers selected from α2-macroglobulin, AST (aspartate aminotransferase), ALT (alanine aminotransferase), GGT (gammaglutamyl transpeptidase), γ-globulin, total bilirubin, albumin, α1-globulin, α2-globulin, haptoglobin, β-globulin, apoA1, IL-10, TGF-β1, ApoA2 and apoB. However, said document does not disclose markers useful to the specific diagnosis of NASH.

Poynard Thierry et al. (Poynard Thierry et al. , BMC Gastroenterology. 2006; 6(1):34) discloses a method of diagnosing NASH/steatosis combining 13 parameters: age, sex, height, weight, and serum levels of triglycerides, cholesterol, alpha 2 macroglobulin, apolipoprotein A1, haptoglobin, gamma-glutamyl-transpeptidase, transaminases ALT, AST and total bilirubin. Said method allows the discrimination between patients having NASH and those having steatosis but no NASH. However, said method does not allow to discriminate between NASH/steatosis and other hepatic diseases.

US 2007/037221 discloses markers and methods to diagnose liver pathology including hepatitis and fatty liver/steatosis. The method disclosed in this document is based on the quantification of the glycosilation on the marker proteins and the comparison of the values obtained for glycosilation of such proteins with reference values in subjects with and/or without liver pathologies.

Santos-Gonzalez et al. (Santos-Gonzalez et al., Experimental Gerontology, 2007, 42 (8):798-806) discloses that the feeding of rats with a Co-enzyme 010-based diet leads to a differential expression of plasma hemopexin, apolipoprotein H, inter-α-inhibitor H4P heavy chain, preprohaptoglobin, Fibrinogen gamma-chain precursor, fetuin-like protein, α-1-antitrypsin precursor, type II peroxiredoxin, serine protease inhibitor 3, vitamin D-binding protein and ApoAl.

The international patent application WO2004/055520 discloses a method of diagnosing non-alcoholic steatohepatitis (NASH) using molecular markers. The method consists of detecting and quantifying, *in vitro* in a hepatic tissue sample, the levels of a protein which can be used as a NASH molecular marker and which is selected from apolipoprotein A1, sub-unit beta of the mitochondrial ATPase, leukotriene A4 hydrolase, keratin 18, guanidine acetate N-methyltransferase, superoxide dismutase, albumin, antioxidant protein 2 (isoform 1), prohibitin 1, methionine adenosyl transferase, long-chain acyl CoA dehydrogenase, selenium binding protein, antioxidant protein 2 (isoform 2), and combination thereof. The invention further consists in comparing the results obtained with the normal values of said proteins in healthy hepatic tissue. Said method can be used to diagnose NASH and/or to assess a subject's potential risk of developing NASH. However, this method comprises a hepatic biopsy and no study has demonstrated that a single or a panel of biomarkers can be used as an alternative to liver biopsy for the diagnosis of NASH.

There is, therefore, a need to develop a diagnosis method that would give a good predictive value of the extent of hepatic steatosis and/or NASH in a subject, and that would be reliable enough to reduce the need of liver biopsy.

### SUMMARY OF THE INVENTION

In one aspect, the invention relates to an *in vitro* method for discriminating between patients suffering NASH or steatosis and patients suffering from other types of liver diseases comprising:
a) detecting and quantifying in a sample: from said subject the levels of antithrombin. III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said proteins and
b) comparing the results obtained in step a) with reference values for levels of said proteins or of the genes encoding said proteins,
wherein
(i) if the levels of antithrombin III and neutrophil cytosolic factor 2 proteins, or of the genes encoding said proteins are lower than reference values for each of said protein, or gene and
(ii) if the levels of glutathione peroxidase 3, acid haptoglobin alpha 2, haptoglobin beta, peroxiredoxin-2, complement factor I, basic haptoglobin alpha 1 and hemoglobin gamma proteins or of the genes encoding said proteins are higher than reference values for each of said proteins, or genes,
then the subject suffers from, NASH or steatosis.

In another aspect, the invention relates to an *in vitro* method for monitoring the effect of the therapy administered to a subject suffering NASH or steatosis, comprising:
a) detecting and quantifying the levels of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said proteins and
b) comparing the results obtained in step (a) with reference values for the levels of said proteins, or genes encoding said proteins,
wherein
(i) if the levels of antithrombin III and neutrophil cytosolic factor 2 proteins, or of the genes encoding said proteins are higher than reference values and
(ii) if the levels of glutathione peroxidase 3, acid haptoglobin alpha 2, haptoglobin beta, peroxiredoxin-2, complement factor I, basic haptoglobin alpha 1 and hemoglobin gamma proteins, or of the genes encoding said proteins are lower than reference values then
the therapy administered to said subject has been effective or is being effective.

In yet another aspect, the invention relates to an *in vitro* method for identifying NASH-and/or steatosis-specific biomarkers comprising:
a) depleting the serum albumin (HSA) contained in a serum sample from a subject suffering from said disease,
b) analyzing the protein profile of the serum sample obtained in step (a) and of a sample,
c) comparing the protein profiles obtained in step (b) with a profile obtained under similar conditions from a control subject and
d) identifying those proteins of the profile obtained from the patient sample which are present at substantially different levels with respect to the profile obtained from the control subject sample,
wherein the proteins identified in step (d) are identified as NASH- and/or steatosis-specific biomarkers.

In another aspect, the invention relates to a kit comprising a set of reagents, wherein said set consists of reagents for detecting antithrombin III, neutrophil cytosolic factor 2, glutathione peroxidase 3, acid haptoglobin alpha 2, haptoglobin beta, peroxiredoxin-2, complement factor I, basic haptoglobin alpha 1, and hemoglobin gamma proteins, or the genes coding said proteins and, optionally, a reagent for detecting a housekeeping protein or the gene coding for said housekeeping protein.

### BRIEF DESCRIPTION OF THE FIGURES

Figure 1 depicts a schematic diagram of the depletion process and images showing a comparison of 2D gels of human serum samples before (A) and after (B) human serum albumin (HSA) depletion by means of an affinity column. Figures in the gel images indicate the position of several markers as follows: 1, albumin, 2, transferrin, 3, Hemopexin (β-1B-glycoprotein), 4, α-1-antitripsin.
Figure 2 is a photograph showing 2D gel images obtained after running the same HSA-depleted NASH serum sample in three independent experiments.
Figure 3 is a graph showing the relative intensity of differential spots (0.67<R<1.5) steatosis/control (S/C) and NASH/control (N/C). Once the control, steatosis and NASH 2D gel images were analysed, 57 differential spots were detected and their intensity values quantified. The values are shown in the figures. The bars whose correspondent protein name is inside a box, correspond to statistically significant (Student's t-test) differential spots with a confidence interval of 95% (p>0.05). The total number of significantly differential spots is 20.

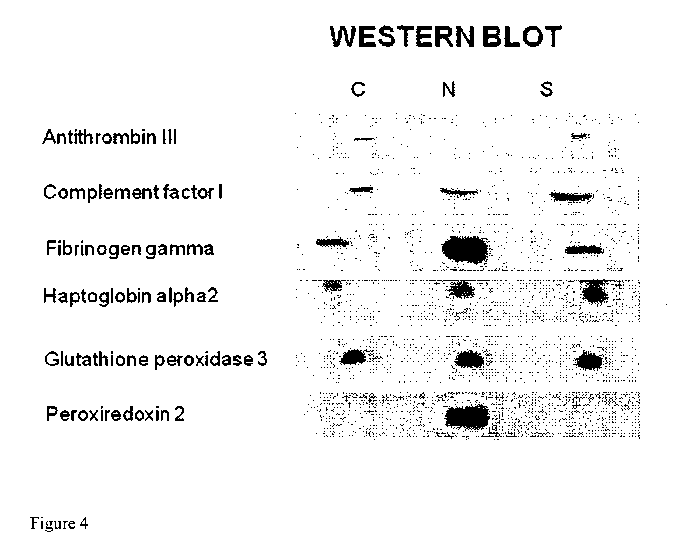

Figure 4 shows 3 examples of western blot analysis of E (steatosis), N (NASH) and C (control) human serum samples probed with antibodies specific for glutathione peroxidasde 3 (upper panel), antithrombin III (middle panel) and complement factor I (lower panel) where the same trend of up- or down- regulation is observed as in the statistical selection of the 2D gel image analysis.

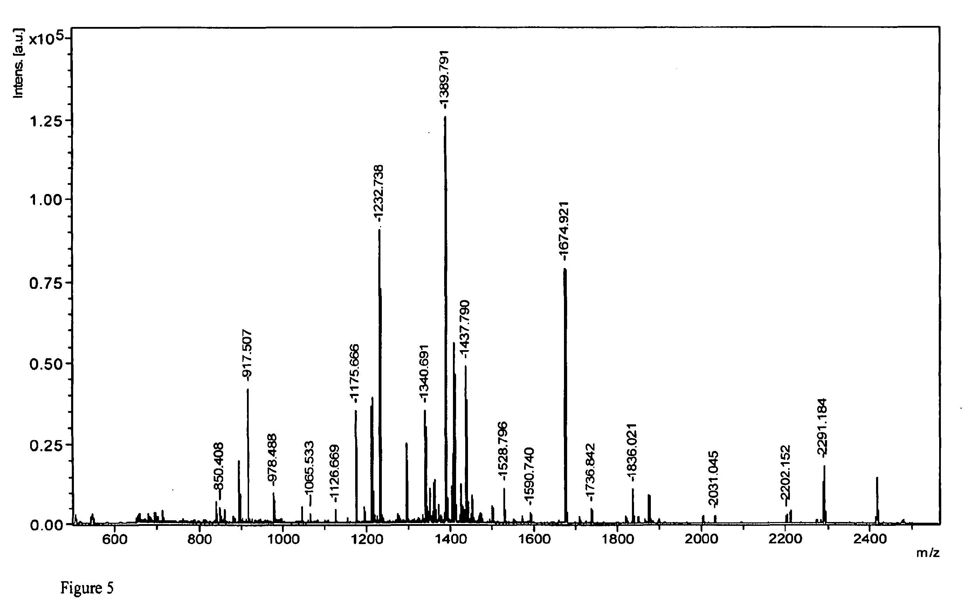

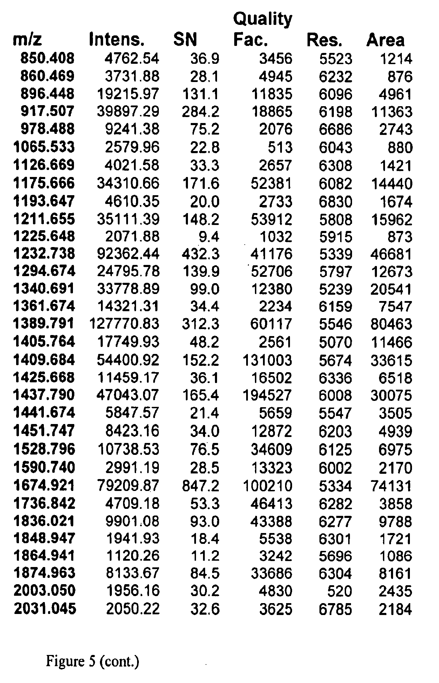

Figure 5 shows the MALDI-TOF mass spectrum of the protein spot showing a molecular weight of 52602 Da and having a pI of 5,25 in 2D gels and which corresponds to antithrombin III.

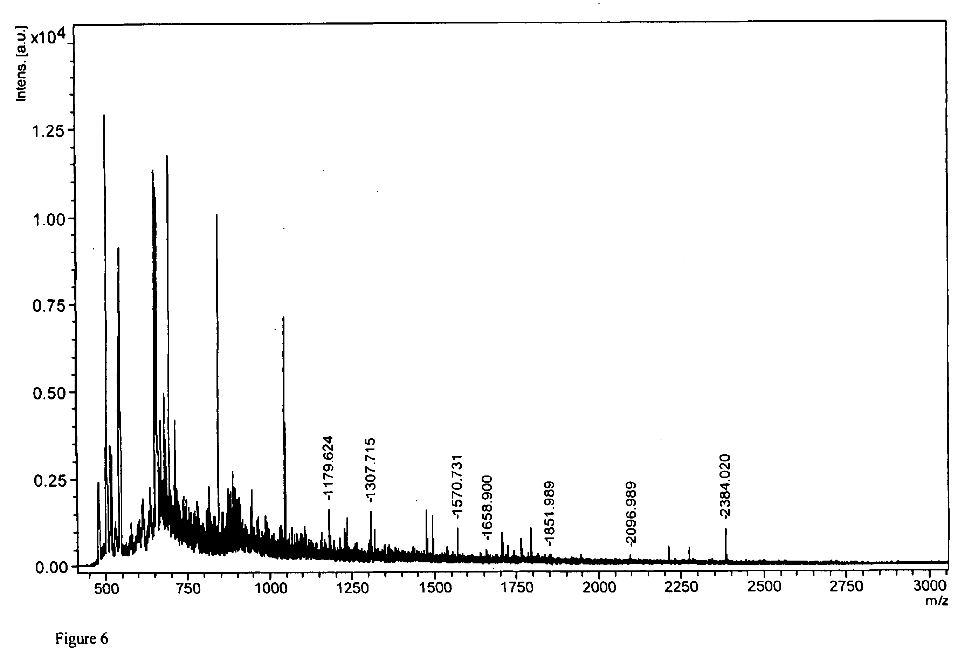

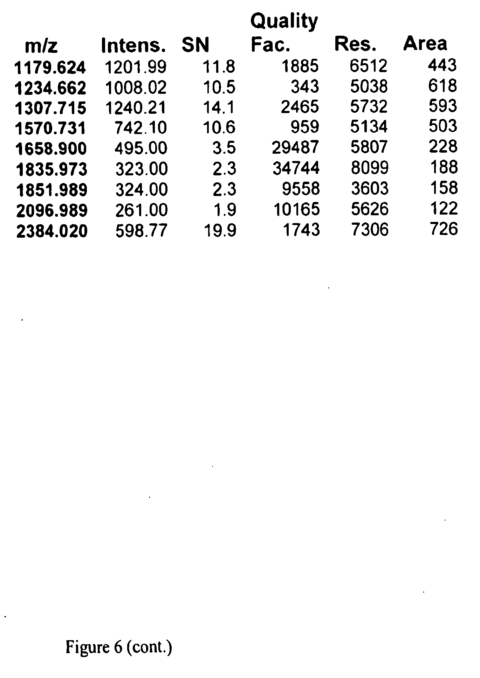

Figure 6 shows the MALDI-TOF mass spectrum of the protein spot showing a molecular weight of 59761 Da and a pI of 5,35 in 2D gels and which corresponds to neutrohil cytosolic factor 2.

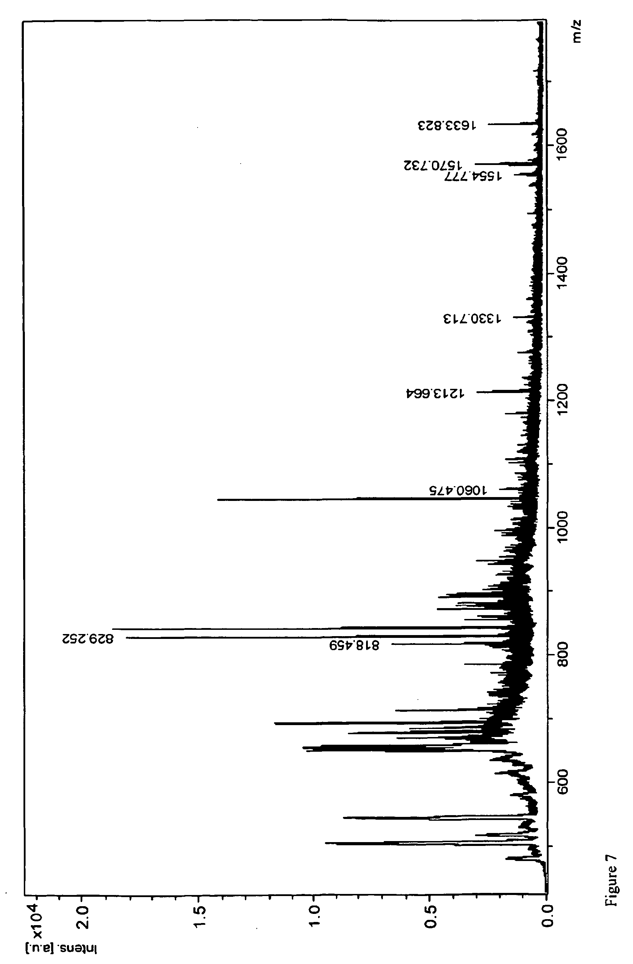

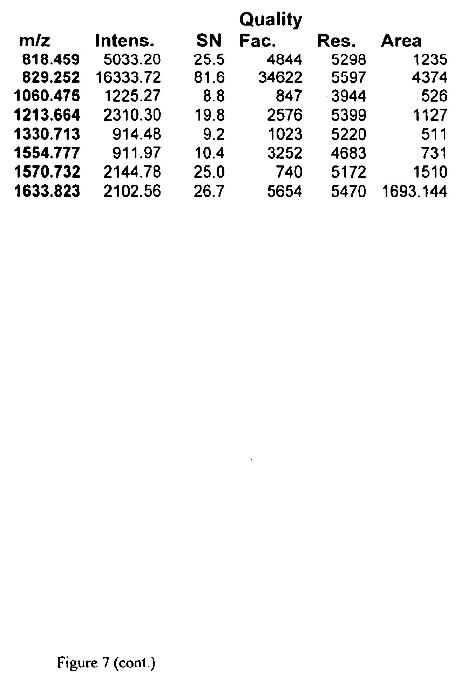

Figure 7 shows the MALDI-TOF mass spectrum of the protein spot showing a molcular weight of 25400 and a pI of 5,56 in 2D gels and corresponding to glutathione peroxidase 3.

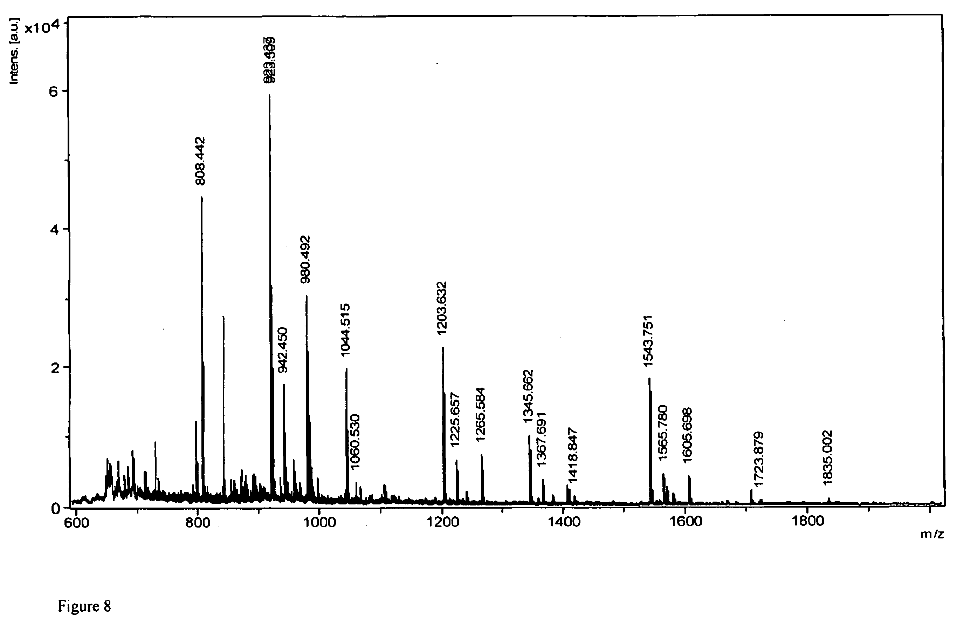

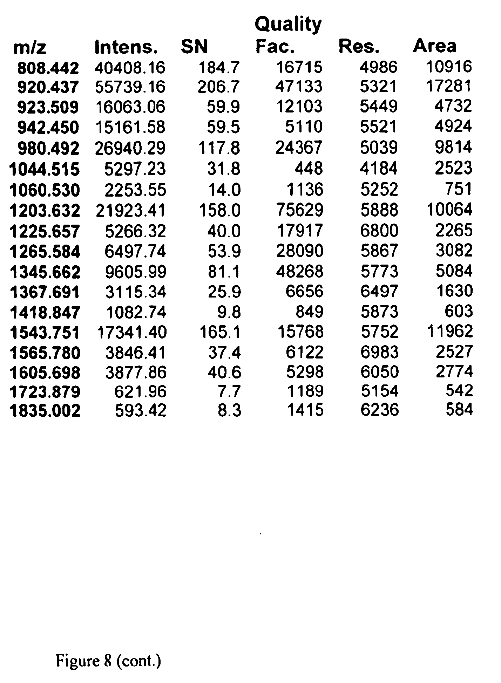

Figure 8 shows the MALDI-TOF mass spectrum of the protein spot showing a molecular weight of 38600 and a pI of 5,38-5,56 and corresponding to haptoglobin beta.

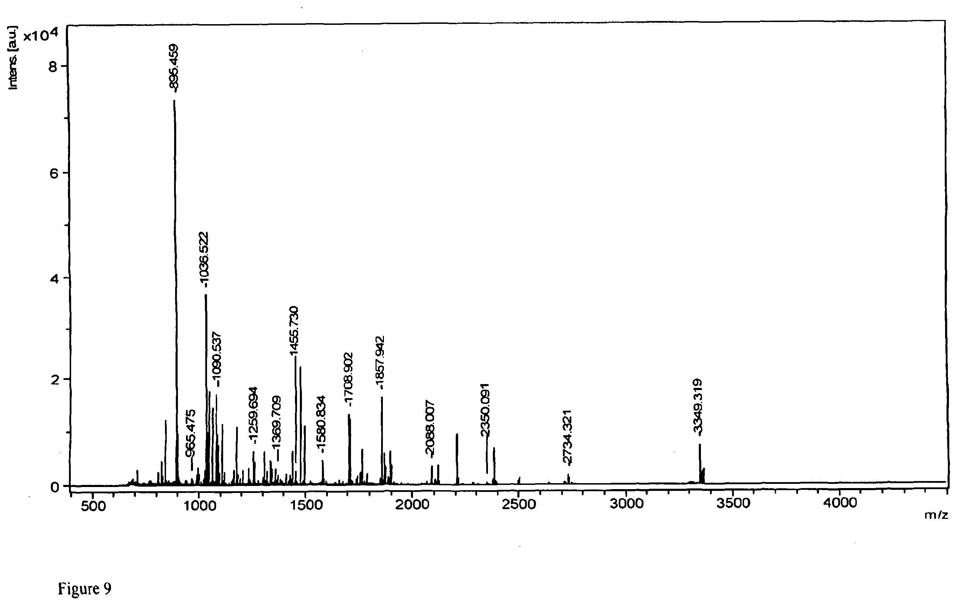

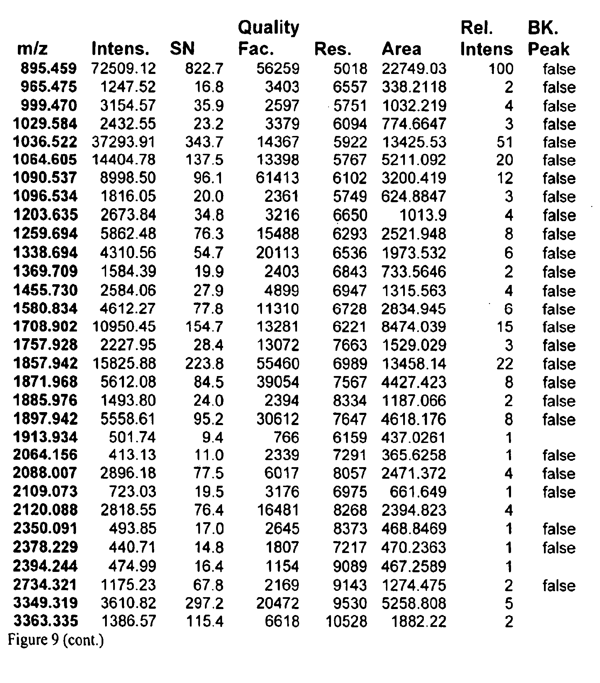

Figure 9 shows the MALDI-TOF mass spectrum of the protein spot showing a molecular weight of 11900 and a pI of 6,61 and corresponding to a basic isoform of haptoglobin alpha 1.

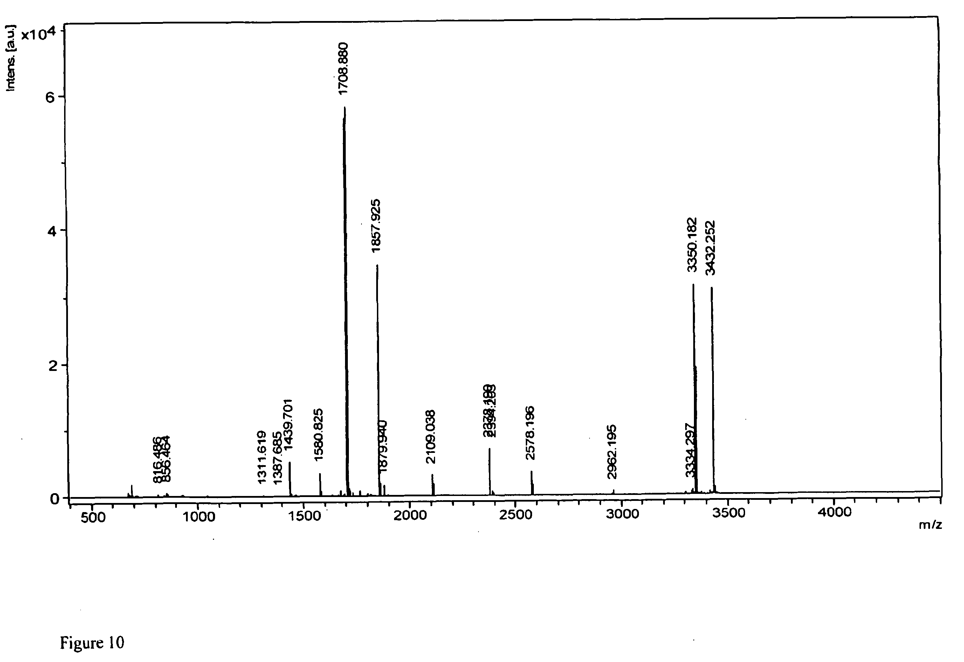

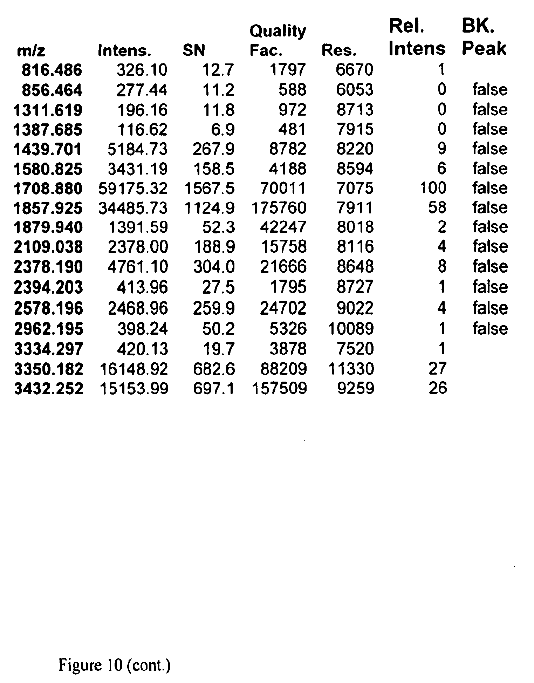

Figure 10 shows the MALDI-TOF mass spectrum of the protein spot showing a molecular weight of 16882 and a pI of 5,4 and corresponding to the acid isoform of the haptoglobin alpha 2 protein.

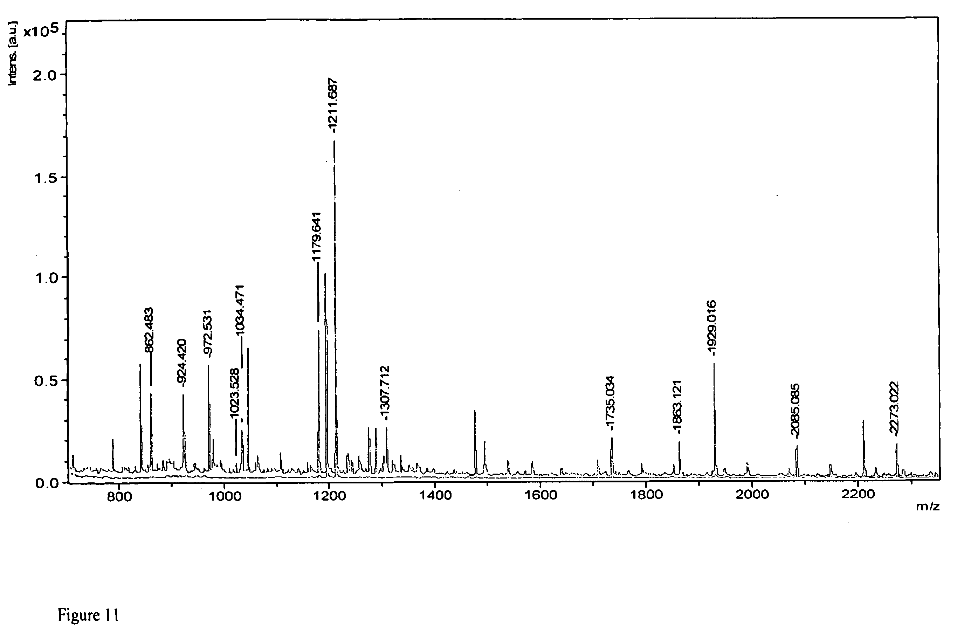

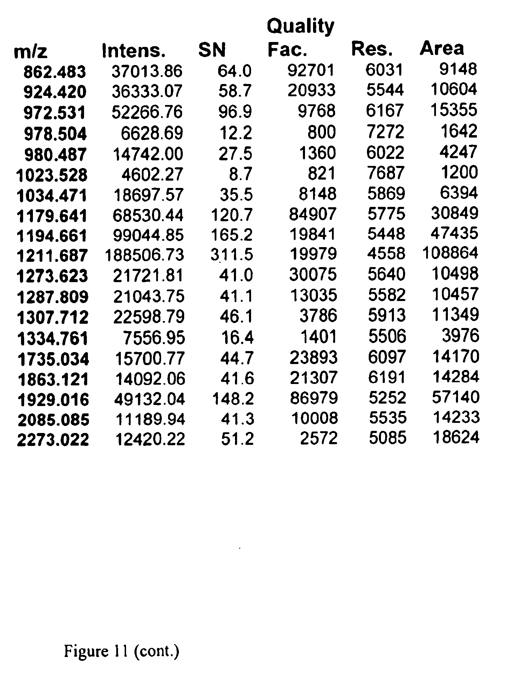

Figure 11 shows the MALDI-TOF mass spectrum of the protein spot showing a molecular weight of 21892 and a pI of 5,48 and corresponding to peroxiredoxin 2.

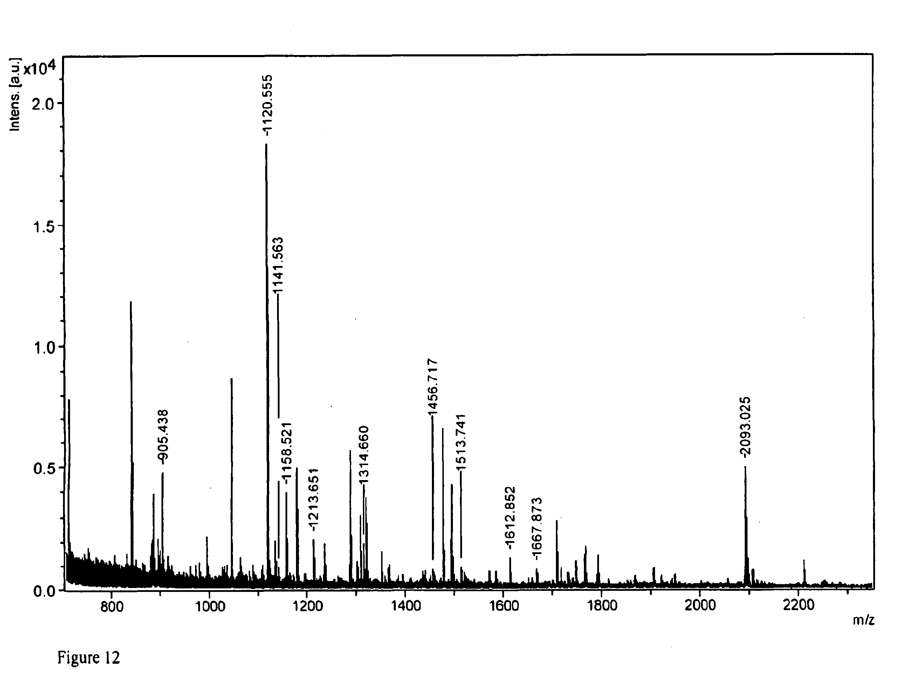

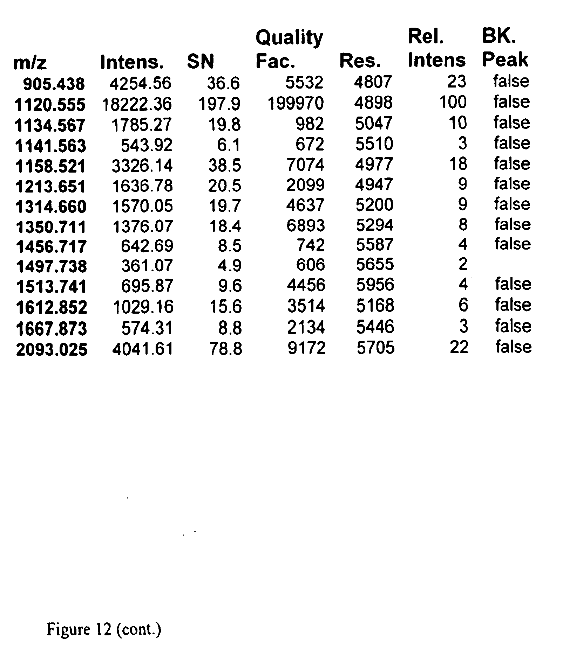

Figure 12 shows the MALDI-TOF mass spectrum of the protein spot showing a molecular weight of 65720 and a pI of 5,68 and corresponding to complement factor I.

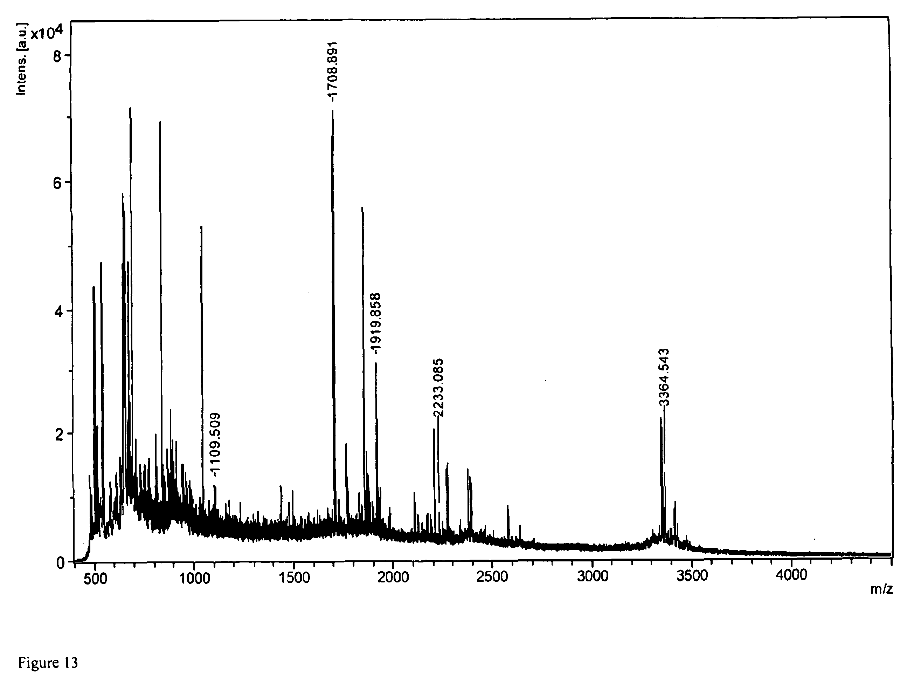

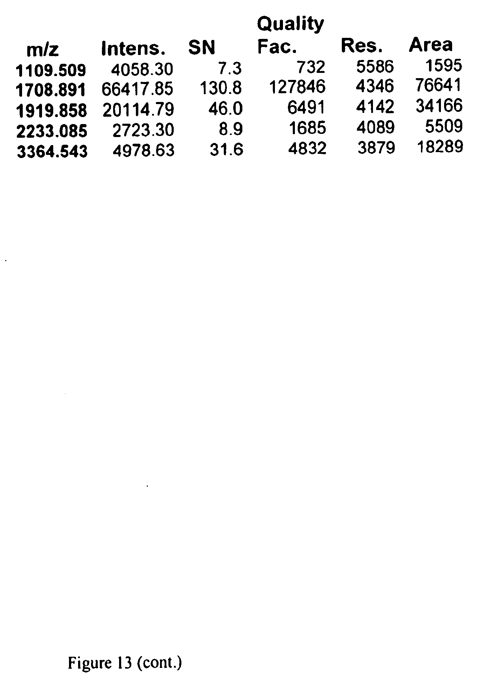

Figure 13 shows the MALDI-TOF mass spectrum of the protein spot showing a molecular weight of 16140 and a pI of 4.71 and corresponding to hemoglobin gamma.

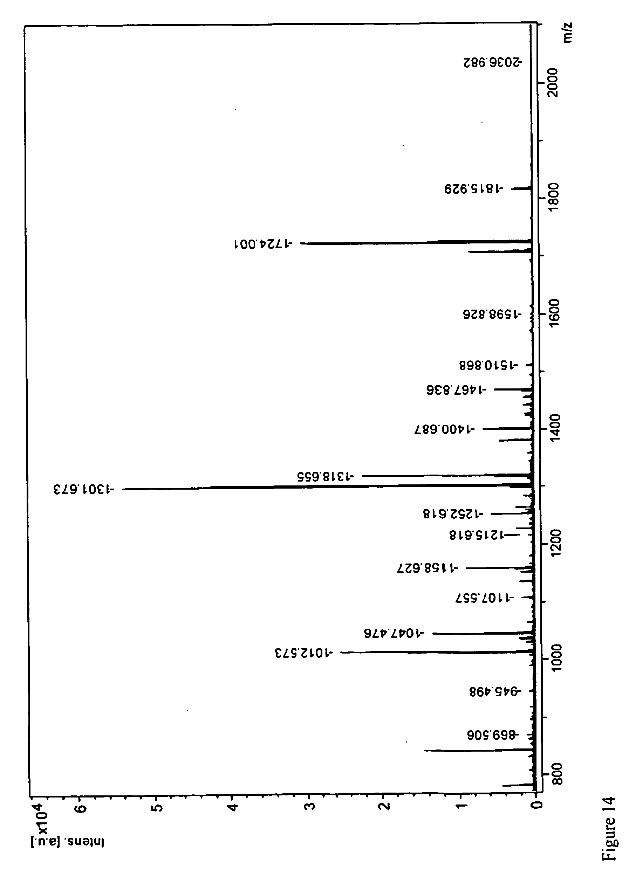

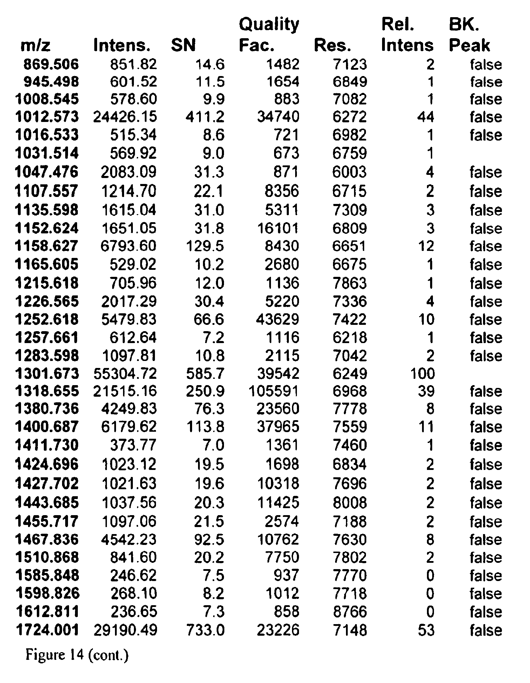

Figure 14 shows a MALDI-TOF mass spectrum of the protein spot showing a molecular weight of 30778 and a pI of 5,48 and corresponding to the apolipoprotein AI.

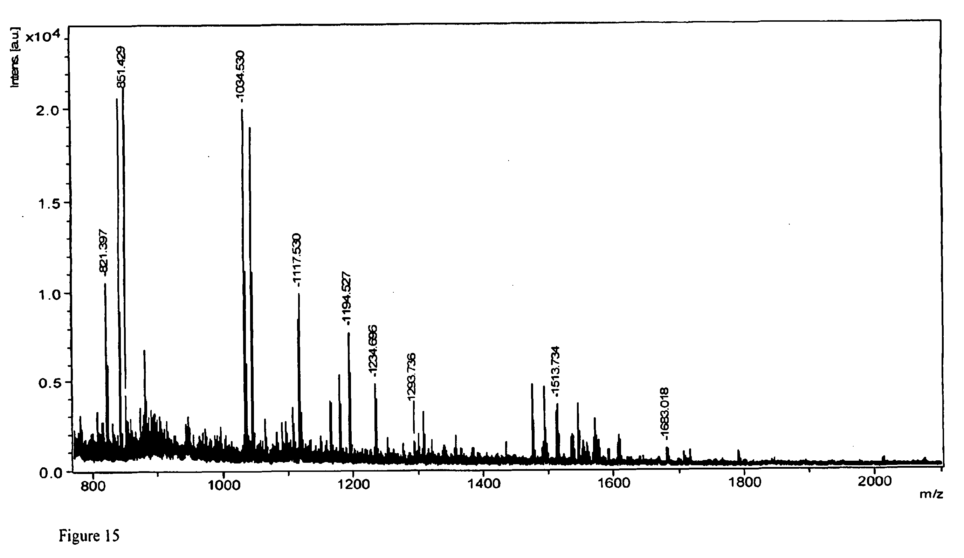

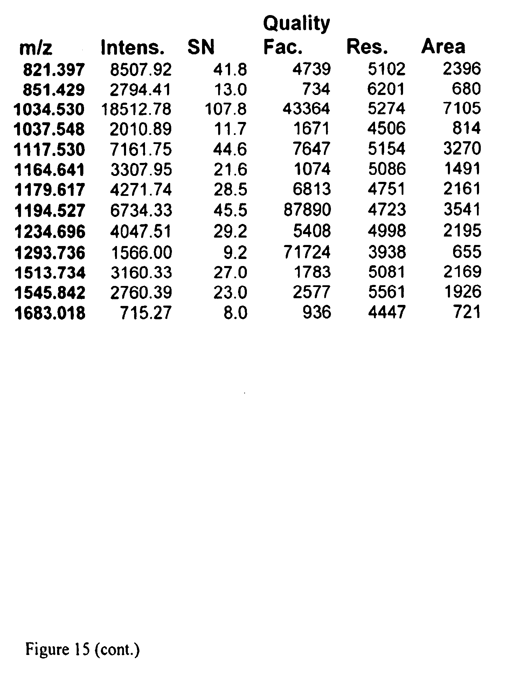

Figure 15 shows the MALDI-TOF mass spectrum of the protein spot showing a molecular weight of 51512 and a pI of 5,03-5,62 and corresponding to the gamma chain of fibrinogen.

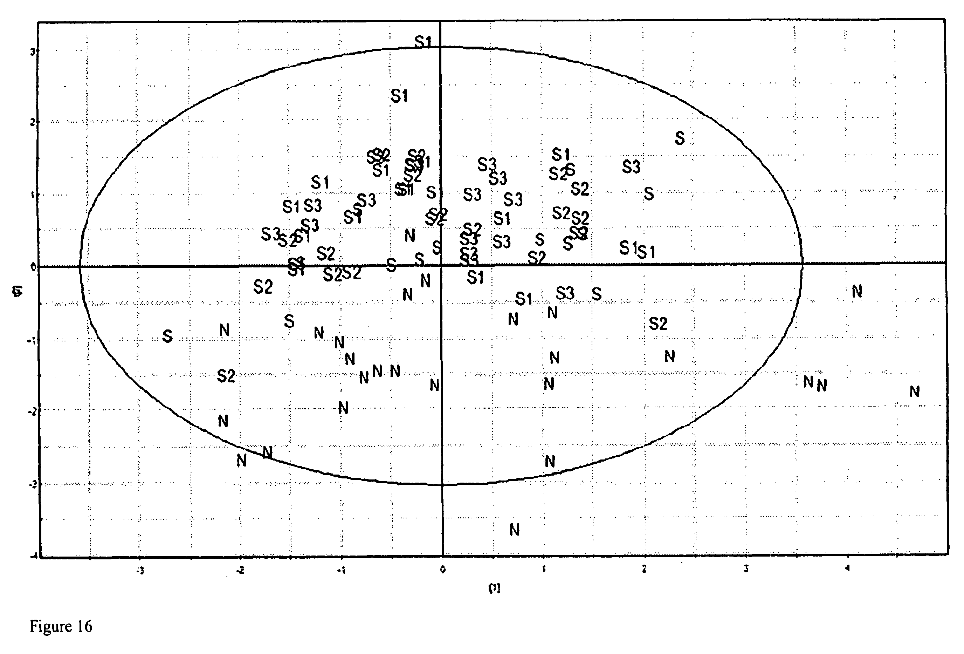

Figure 16: Principal Component Analysis (PCA) of ELISA absorbance values. Data from ELISA assays for 7 proteins (Haptoglobin, Complement Factor I, Antithrombin III, Apolipoprotein A1, Glutathione peroxidase 3, Peroxiredoxin 2 and Neutrophil cytosolic factor 2) selected from the list of 9 candidate biomarkers were normalized according to serum total protein concentration of each sample. Data were obtained from serum samples of 26 NASH (N) patients and 69 steatosis patients with a different degree of steatosis (ranging from S to S3). All of the samples were diagnosed by liver biopsy.

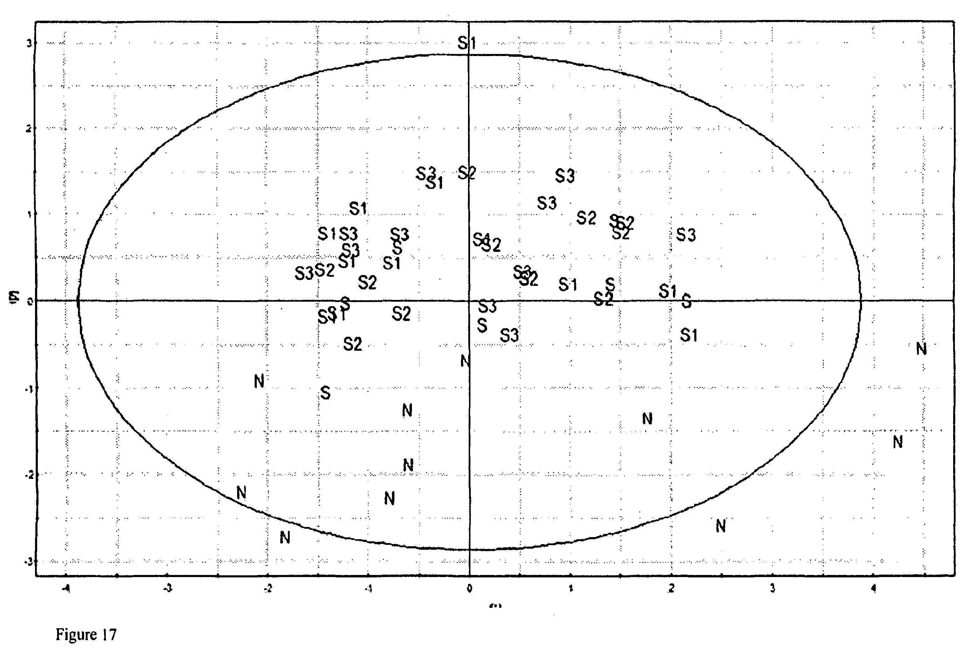

Figure 17: Principal Component analysis (PCA) ELISA absorbance values for serum samples from female patients. Data from ELISA assays for 7 proteins (same as in figure 16) were normalized according to serum total protein concentration of each sample. Data were obtained from Serum samples of 11 female NASH patients (N) and 41 steatosis female patients with a different degree of steatosis (ranging from S to S3).

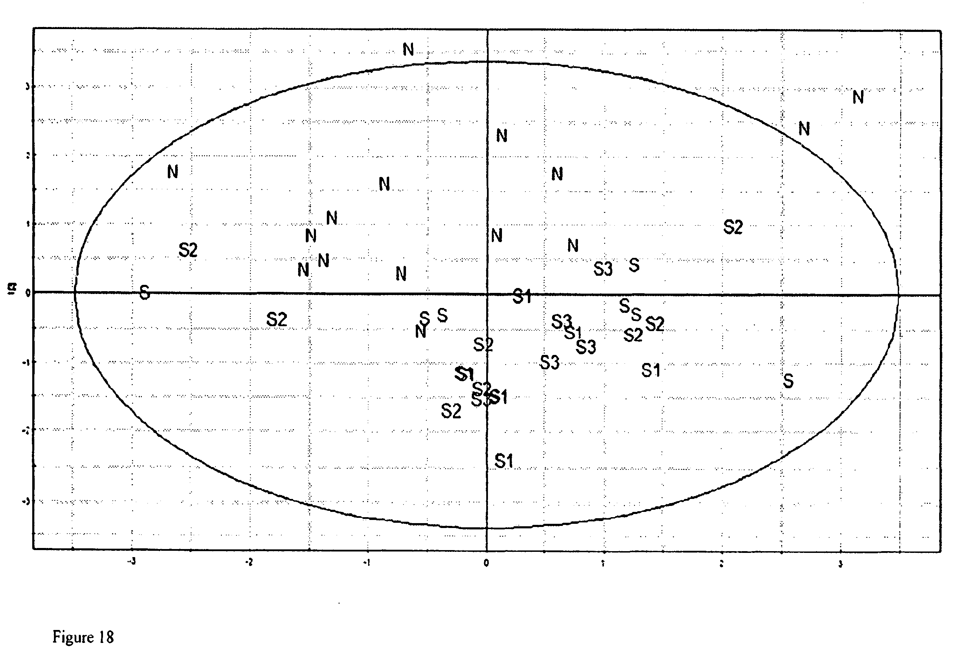

Figure 18: Principal Component analysis (PCA) ELISA absorbance values for serum samples from male patients Data from ELISA assays for 7 proteins (same as in figure 16) were normalized according to serum total protein concentration of each sample. Data were obtained from Serum samples of 15 male Nash patients (N) and 28 steatosis male patients with a different degree of steatosis (ranging from S to S3).

### DETAILED DESCRIPTION OF THE INVENTION

The inventors of the present invention have discovered a new set of molecular markers present in plasma and/or serum which, surprisingly, allows detecting and/or diagnosing not only NASH but also steatosis and, more importantly, allows to discriminate between patients suffering NASH or steatosis and patients suffering from other types of liver diseases such as hepatitis B, hepatitis C, autoimmune hepatitis or primary biliary cirrhosis. The new set of molecular markers is based on nine proteins giving rise to a set of eleven biomarkers (hereinafter each biomarker is considered as a protein) which, depending on its expression level, will be or not indicative of NASH and/or steatosis. The new set of molecular markers comprises the following proteins: antithrombin III, neutrophil cytosolic factor 2, glutathione peroxidase 3, acid haptoglobin alpha 2, haptoglobin beta, peroxiredoxin-2, complement factor I, basic haptoglobin alpha 1, hemoglobin gamma, apolipoprotein AI and fibrinogen gamma proteins. The molecular weighs of said proteins are shown in Table 1.

| Protein | | MW (Da) | pI |
|---|---|---|---|
| Antithrombin III | | 52602 | 5,25 |
| Neutrophil cytosolic factor 2 | | 59761 | 5,35 |
| Glutathione peroxidase 3 | | 25402 | 5,56 |
| Haptoglobin full-length | | 45205 | |
| | beta | 38600 | 5,38-5,56 |
| | alpha 1 (basic) | 11900 | 6,61 |
| | alpha 2 (acidic) | 16882 | 5,4 |
| Peroxiredoxin 2 | | 21892 | 5,48 |
| Complement component 1 | | 65720 | 5,68 |
| Hemmoglobin gamma | | 16140 | 4,71 |
| Apolipoprotein A1 | | 30778 | 5,48 |
| Fibrinogen, gamma chain | | 51512 | 5,03-5,62 |

| | | | |
|---|---|---|---|
| Basic and acid refer, respectively, to a basic isoform of haptoglobin alpha 1 (Isoelectric point=6.61) and an acid isoform of haptoglobin alpha 2 (isoelectric point= 5.4) as both chains (alpha1 and alpha 2) present several different isoforms. | | | |

Thus, the invention relates to an *in vitro* method for discriminating between patients suffering NASH or steatosis and patients suffering from other types of liver diseases comprising:
a) detecting and quantifying in a sample from said subject the levels of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said proteins and
b) comparing the results obtained in step a) with reference values for levels of said proteins or of the genes encoding said proteins,
wherein
(i) if the levels of antithrombin III and neutrophil cytosolic factor 2 proteins, or of the genes encoding said proteins are lower than reference values for each of said protein, or gene and
(ii) if the levels of glutathione peroxidase 3, acid haptoglobin alpha 2, haptoglobin beta, peroxiredoxin-2, complement factor I, basic haptoglobin alpha 1 and hemoglobin gamma proteins or of the genes encoding said proteins are higher than reference values for each of said proteins, or genes,
then the subject suffers from, NASH or steatosis.

Moreover, the invention contemplates the use of each of the above mentioned polypeptides as biomarkers for the diagnosis of NASH or for the identification of subjects with predisposition to suffer NASH. Thus, the invention contemplates in vitro methods for the diagnosis of NASH and/or steatosis or for the identification of subjects which are predisposed to develop NASH and/or steatosis comprising determining the levels of at least one protein or variant thereof wherein the protein is selected from the group of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma wherein
(i) if the levels of antithrombin III or of the genes encoding said protein are lower than reference values for each of said protein, or gene or
(ii) if the levels of neutrophil cytosolic factor 2 or of the genes encoding said protein are lower than reference values for each of said protein, or gene or
(iii) if the levels of glutathione peroxidase 3 or of the genes encoding said protein are higher than reference values for each of said protein, or gene or
(iv) if the levels of acid haptoglobin alpha 2 or of the genes encoding said protein thereof are higher than reference values for each of said protein, or gene or
(v) if the levels of acid haptoglobin beta or of the genes encoding said protein are higher than reference values for each of said protein, or gene or
(vi) if the levels of acid peroxiredoxin-2 or of the genes encoding said protein are higher than reference values for each of said protein, or gene or
(vii) if the levels of complement factor I or of the genes encoding said protein are higher than reference values for each of said protein, or gene or
(viii) if the levels of basic haptoglobin alpha 1 or of the genes encoding said protein are higher than reference values for each of said protein, or gene or
(ix) if the levels of hemoglobin gamma or of the genes encoding said protein are higher than reference values for each of said protein, or gene then
the subject suffers from NASH or steatosis or is predisposed to developing NASH or steatosis.

The term "subject", as used herein, refers to all animals classified as mammals and includes, but is not restricted to, domestic and farm animals, primates and humans, e.g., human beings, non-human primates, cows, horses, pigs, sheeps, goats, dogs, cats, or rodents. Preferably, the subject is a male or female human of any age or race. Illustrative, non limitative examples of subjects according to the first method of the invention include subjects either suspected of having fatty infiltration of the liver, or subjects with serum aminotransferase elevations for a long period (over 6 months' duration) or subjects suspected of having NASH and/or steatosis, or subjects previously diagnosed or not with NASH and/or steatosis, or subjects who are receiving or have previously received anti-NASH treatment of anti-steatosis treatment, or subjects having normal hepatic function tissue, i.e., a tissue determined, by one of ordinary skill in the medical art, to have no evidence of fatty infiltration of the liver, often accompanied by hepatocellular damage with inflammation. Methods for identifying subjects suspected of having NASH and/or steatosis may include physical examination, subject's family medical history, subject's medical history, liver biopsy, or a number of imaging technologies such as ultrasonography.

The term "NASH" refers to non-alcoholic steatohepatitis. Diagnostic methods for NASH and the clinical delineation of NASH diagnoses are well known to those skilled in the medical arts.

The term "steatosis" (also called fatty change, fatty degeneration or adipose degeneration) refers to the process describing the abnormal retention of lipids within a cell.

The expressions "higher expression levels" and "lower expression levels" of a protein in a sample compared to reference values for said protein, as used herein, are understood as statistically significant differences in the expression levels of a protein in the sample under study in comparison with a reference value for the expression levels of said protein. The expression levels in the protein are understood as being higher than a reference value for said protein if the differences in expression levels are at least 5%, 10% or 20%, more preferred at least 50% or may even be as high as 75% or 100% than the reference value. More preferred the difference in the level of expression is at least 200%, i.e. two fold, at least 500%, i.e. five fold, or at least 1000%, i.e. 10 fold in the test sample when compared with the reference value.

Similarly, the expression levels of the protein under study are understood as being lower than the reference values for said protein if the expression levels of the protein under study are at least 5%, 10% or 20%, more preferred at least 50% or may even be as low as 75% or 100% than in the reference value. More preferred the difference in the level of expression is at least 200%, i.e. two fold, at least 500%, i.e. five fold, or at least 1000%, i.e. 10 fold lower in the first sample when compared to the reference value.

The determination of the levels of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said polypeptides allows the discrimination of patients suffering from NASH or steatosis from patients suffering other liver diseases (e.g. hepatitis A, hepatitis C, primary biliar cirrhosis, autoimmune hepatitis or Wilson syndrome) but does not allow to further discriminate NASH patients from steatosis patients. For this purpose, the signature must be complemented with a further biomarker. In particular, the inventors have also discovered that apolipoprotein A1 is an additional differentially expressed protein which, when combined with the rest of the markers, allows the diagnosis of steatosis without giving NASH patients as false positives. Moreover, the inventors have also discovered that fibrinogen gamma is an additional differentially expressed protein which, when combined with the rest of the markers, allows the diagnosis of NASH without giving steatosis patients as false positives.

Thus, in a particular embodiment, the first method of the invention further comprises detecting and quantifying apolipoprotein A1 protein, or the gene coding said protein, wherein if apoliprotein A1 protein, or the gene coding said protein, is down-regulated compared to reference values, then the subject suffers from, or is predisposed to develop steatosis.

Likewise, in another particular embodiment, the first method of the invention further comprises detecting and quantifying fibrinogen gamma protein, or the gene coding said protein, wherein if fibrinogen gamma protein, or the gene coding said protein, is down-regulated compared to reference values, then the subject suffers from, or is predisposed to develop NASH.

Moreover, the additional biomarkers which allow to discriminate between NASH and steatosis can also be used as isolated biomarkers for the diagnosis of NASH/steatosis or for the identification of subjects with predisposition to suffer NASH/steatosis. Thus, the invention contemplates *in vitro* methods for the diagnosis of NASH and/or steatosis or for the identification of subjects which are predisposed to develop NASH and/or steatosis comprising determining the levels of at least one protein or variant thereof wherein the protein is selected from the group of apolipoprotein A1 and fibrinogen gamma wherein
(i) if the levels of apolipoprotein A1 or of the genes encoding said protein are lower than reference values for said protein, or gene then the subject suffers from steatosis or is predisposed to developing steatosis or
(ii) if the levels of fibrinogen gamma or of the genes encoding said protein are lower than reference values for said protein, or gene then the patient the subject suffers from NASH or is predisposed to developing NASH.

As known in the art the "similarity" between two proteins is determined by comparing the amino acid sequence and its conserved amino acid substitutes of one protein to a sequence of a second protein. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIGH Bethesda, Md. 20894, Altschul, S., et al., J. Mol. Biol. 215: 403-410 (1990)].

The proteins of the invention can be post-translationally modified. For example, post-translational modifications that fall within the scope of the present invention include signal peptide cleavage, glycosylation, acetylation, isoprenylation, proteolysis myristoylation, protein folding and proteolytic processing, etc. Additionally, the proteins may include unnatural amino acids formed by post-translational modification or by introducing unnatural amino acids during translation.

In the context of the present invention, the expression of a protein or of a gene coding said protein is "down- regulated" when the expression level of said protein or gene is decreased
compared to reference values.

In the context of the present invention, the expression of a protein or of a gene coding said protein is "up- regulated" when the expression level of said protein or gene is increased compared to reference values.

In the context of the first method of the invention, "reference values" is understood as the level values of the same proteins (or the genes coding said proteins) in biological samples from control subjects, i.e. healthy subjects or subjects without a clinical history of non-alcoholic fatty liver disease (NAFLD). Moreover, the authors of the present invention have also observed that the biomarker signature described herein allows the discrimination between steatosis and NASH and other liver diseases. Thus, samples from patients suffering from said other liver diseases (hepatitis C, hepatitis B, primary biliar cirrhosis, autoimmune hepatitis and Wilson syndrome) can also be used as control samples to obtain reference values for comparing with the values of the patients under study.

In order to carry out the first method of the invention, a sample is obtained from the subject under study. The term "sample" as used herein, relates to any sample which can be obtained from a subject. The present method can be applied to any type of biological sample from a subject, such as a biopsy sample, tissue (liver tissue), cell or fluid (serum, saliva, semen, sputum, cerebral spinal fluid (CSF), tears, mucus, sweat, milk, brain extracts and the like).

In a particular embodiment, the sample is serum or plasma which can be obtained by conventional methods. In another particular embodiment, the sample is HSA depleted serum or plasma. The plasma or serum may be utilized directly for identification of the expression levels of the proteins, or the nucleic acid is extracted from said plasma or serum in order to determine the expression levels of the genes coding said proteins.

The determination of the expression levels of the proteins can be carried out by immunological techniques such as ELISA, Western Blot or immunofluorescence. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry or fluorometry. Thus, in a preferred embodiment, the proteins are detected by western blot, ELISA, a protein array or a two-dimensional electrophoresis. The skilled person will appreciate that, if the proteins forming the NASH- or steatosis-specific signature are to be detected by two-dimensional electroforesis, the two-dimensional gel must be stained in order to detect the polypeptides. Any staining method which is sensitive enough for detecting the proteins forming the signature can be used and includes, without limitation, silver staining, Coomassie Blue staining, Sypro Ruby staining, squarylium dyes staining, fluorescent cyanine dyes staining as described in Mujumdar, R. B. et al., (Cytometry, 1989, 10:11-19 and US5268486), dipyrromethene boron difluoride dyes staining as described in US4774339 and the like.

Once the spots showing statistically significant differences between the two conditions are identified, the proteins forming the signature are identified based on their molecular weight and isoelectric point. The identity of the protein spots found in the two-dimensional gel can be confirmed by cutting out the spot, performing an in-gel digestion and subsequent mass-spectrometry. The levels of the proteins in the selected spots can be quantified using any known technique such as densitometry of the stained gels and volume (intensity x height) determination, differential in-gel electrophoresis (DIGE) wherein the proteins to be separated are labelled with fluorophores prior to their electrophoretic fractionation and are then mixed with an internal standard representing all proteins in the sample and which can be used for normalisation purposes.

The regions of the gels where the differentially-expressed protein is found can be excised and the identity of the protein spot confirmed by direct sequencing using techniques such as direct mass determination (MALI-MS) or peptide ladder sequencing (MALDI-MS/ESI-MS). Alternatively, the protein spots can be digested with any suitable protease or chemical reagent and the resulting peptides identified using a technique selected from nano-spray ESI-MS/MS, peptide mass profiling by ESI-MS, peptide mass profiling by MALDI-MS, internal sequencing by Edman degradation or N-terminal sequencing. Protein mixtures can also be analyzed without prior separation. These procedures begin with proteolytic digestion of the proteins in a complex mixture. The resulting peptides are often injected onto a high pressure liquid chromatography column (HPLC) that separates peptides based on hydrophobicity. HPLC can be coupled directly to a time-of-flight mass spectrometer using electrospray ionization. Peptides eluting from the column can be identified by tandem mass spectrometry (MS/MS). Labelling with isotope tags can be used to quantitatively compare proteins concentration among two or more protein samples.

In a preferred embodiment, the analysis of the protein sample is carried out by in-gel tryptic digestion followed by MALDI-TOF.

When an immunological method is used, any antibody or reagent known to bind with high affinity to the target proteins can be used for detecting the amount of target proteins. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

On the other hand, the determination of the protein expression levels can be carried out by constructing a tissue microarray (TMA) containing the subject samples assembled, and determining the expression levels of the proteins by immunohistochemistry techniques well known in the state of the art.

Alternatively, the first method of the invention can be put into practice determining the expression levels of the gene coding the cited proteins or the variants thereof The determination of the expression levels of a gene can be carried out by measuring the expression levels of the mRNA from said gene. For this purpose, the sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art [Sambrook, J., et al., 2001. Molecular cloning: a Laboratory Manual, 3rd ed., Cold Spring Harbor Laboratory Press, N.Y., Vol. 1-3]. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (RT-PCR, SAGE, or TaqMan) are suitable for use in performing the foregoing method of the invention, the gene mRNA expression levels are often determined by reverse transcription polymerase chain reaction (RT-PCR).

In the context of the present invention, the set of biomarkers herein disclosed can be used for monitoring the effect of the therapy administered to a subject suffering NASH and/or steatosis who has previously received anti-NASH treatment and/or anti-steatosis treatment.

Thus, in another aspect, the invention relates to an *in vitro* method (hereinafter second method of the invention) for monitoring the effect of the therapy administered to a subject suffering NASH or steatosis, comprising:
a) detecting and quantifying the levels of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said proteins and
b) comparing the results obtained in step (a) with reference values for the levels of said proteins or genes encoding said proteins ,
wherein
(a) if the levels of antithrombin III and neutrophil cytosolic factor 2 proteins
   or of the genes encoding said proteins are higher than reference values and
(b) if the levels of glutathione peroxidase 3, acid haptoglobin alpha 2, haptoglobin beta, peroxiredoxin-2, complement factor I, basic haptoglobin alpha 1 and hemoglobin gamma proteins or of the genes encoding said proteins are lower than reference values then
the therapy administered to said subject has been effective or is being effective.

The skilled person will appreciate that reference values, as understood in the context of the second method of the invention, relates to values of the levels of the expression of the different proteins, or genes encoding said proteins in a subject which is suffering from NASH or steatosis and which has been treated with a control compound or which has not been treated.

In a particular embodiment, the second method on the invention further comprises detecting and quantifying apolipoprotein A1 protein or the gene coding said protein, wherein if apoliprotein A 1 protein or the gene coding said protein, is down-regulated compared to reference values, then the therapy administered to said subject has not been effective or are not being effective for the treatment of esteatosis.

In another particular embodiment, the second method of the invention further comprises detecting and quantifying fibrinogen gamma protein or the gene coding said protein, wherein if fibrinogen gamma protein or the gene coding said protein, is down-regulated compared to reference values, then the therapy administered to said subject has not been effective or are not being effective for the treatment of NASH.

Moreover, the invention contemplates the use of the each of the different markers for monitoring the effect of the therapy administered to a subject suffering NASH or steatosis. Thus, the invention contemplates in vitro methods for the monitoring the effect of a therapy administered to a subject suffering from NASH or steatosis comprising determining the levels of at least one protein wherein the protein is selected from the group of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma wherein
(i) if the levels of antithrombin III or of the genes encoding said protein are higher than reference values for each of said protein, or gene or
(ii) if the levels of neutrophil cytosolic factor 2 or of the genes encoding said protein are higher than reference values for each of said protein, or gene or
(iii) if the levels of glutathione peroxidase 3 or of the genes encoding said protein thereof are lower than reference values for each of said protein, or gene or
(iv) if the levels of acid haptoglobin alpha 2 or of the genes encoding said protein are lower than reference valuesfor each of said protein, or gene or
(v) if the levels of acid haptoglobin beta or of the genes encoding said protein are lower than reference values for each of said protein, or gene or
(vi) if the levels of acid peroxiredoxin-2 or of the genes encoding said protein are lower than reference values for each of said protein, or gene or
(vii) if the levels of complement factor I or of the genes encoding said protein are lower than reference values for each of said protein, or gene or
(viii) if the levels of basic haptoglobin alpha 1 or of the genes encoding said protein are lower than reference values for each of said protein, or gene or
(ix) if the levels of hemoglobin gamma or of the genes encoding said protein are lower than reference values for each of said protein, or gene then
then the therapy administered to said subject has been effective or is being effective. The "reference valules" correspond to those of the expression levels of patients suffereing from NASH or steatosis and which have not been treated or have been treated with a placebo.

The terms "NASH", "steatosis", "sample", "subject", "down-regulated" and "up-regulated" have been already defined and can be applied to the present method.

In a particular embodiment of the second method of the invention, the sample is serum or plasma, preferably, HSA-depleted serum or plasma

In the context of the second method of the invention, "reference values" is understood as the levels of the proteins according to the invention (or the genes coding said proteins) in a biological sample from a subject suffering NASH and/or steatosis who has previously received anti-NASH treatment and/or anti-steatosis treatment.

As the skilled person understands, all the methods and techniques previously cited for determining the protein and gene expression levels can be also used in the second method of the invention.

In another particular embodiment, the detection of the proteins is carried out by western blot, ELISA, a protein array or a two-dimensional electrophoresis.

It is also disclosed an *in vitro* method for identifying NASH- or steatosis-specific biomarkers comprising:
a) depleting the human serum albumin (HSA) contained in a serum sample from a subject suffering from said disease,
b) analyzing the protein composition of the serum sample obtained in step (a),
c) comparing the protein composition obtained in step (b) with the protein composition obtained from a control subject, and
d) identifying those proteins which are expressed in the protein composition obtained in step (b) at different levels than in the protein composition obtained from the control subject,
wherein the identified proteins are NASH- or steatosis-specific specific biomarkers.

In step (a), the method comprises depleting the HSA from the serum. A classical depletion strategy for albumin involves the use of the hydrophobic dye Cibacron blue, a chlorotriazine dye which has high affinity for albumin, although using antibody affinity ligands for HSA results in more specific depletion compared to the traditional Cibacron blue depletion method. Affinity media are made up of matrices with covalently attached antibodies to the specific protein. Inmunoaffinity media for HSA depletion is commercially available, packed as spin columns that are compatible with centrifugation. On the other hand, there are kits commercially available for depleting HSA from serum and plasma samples, such as Vivapure anti-HSA kit from Sartorius, Göttingen, Germany

In step (b), the method comprises analyzing the protein composition of the HSA-depleted serum sample from the subject. As the skilled person understands, multiple approaches can be used to identify the protein composition of a sample and, in particular, of the serum. In one possible method it is disclosed that the proteins in the HSA-depleted sample are fractionated using gel electrophoresis. In another method it is disclosed that the gel electrophoresis is a two-dimensional gel electrophoresis. Other methods for the characterisation of the proteins present in a protein sample involve peptide mass fingerprinting (PMF) is a sensitive protein identification technique that compares the masses of experimentally obtained peptides to theoretical digests of proteins available in databases. More recently, multidimensional protein identification technology (MUDPIT) or "shotgun proteomics" has been developed. This technique differs from PMF in that the entire mixture is digested before separation and the resulting peptide mixture is processed by using 2D liquid chromatography and tandem mass spectrophotometry (MS/MS). The peptide sequences are predicted from the observed collision-induced dissociation (CID) mass spectra by comparison with published databases. It is disclosed that the serum is first subjected to an electrophoresis (e.g. two-dimensional electrophoresis) in order to separate the proteins and after the proteins are identify using mass spectrophotometer (see Example 1). Methods and conditions to carry out electrophoresis and mass spectrophotometer technique are widely known from the state of the art.

Once the protein composition of the serum is known, step (c) comprises comparing the protein profile obtained in step (b) with the protein profile obtained from a control subject under similar conditions (i.e., preferably depleted from serum albumin in parallel with the test sample). In the context of the above method a "control subject" is understood as a healthy subject or a subject without a clinical history of NASH and/or steatosis. The comparison step can be carried out by visual inspection of the different protein profiles. Alternatively, if the protein profile has been obtained by two-dimensional gel electrophoresis, the protein profiles can be compared using the PDQuest software.

Last, step (d) of the disclosed method include the identification of proteins which are differentially expressed between the protein profiles obtained in the test sample and the protein profiles of the control sample. The identification of those proteins which are differentially expressed in the NASH/steatosis sample with respect to the control samples is carried out using standard techniques and includes both direct N-terminal or sequencing by Edman degradation or mass spectrometry of a proteolytic digestion of the protein. The protein can be digested with a protease or chemical reagent of choice directly in-gel or can be electro-eluted and then digested in the test tube. The peptides resulting from the digestion can be directly analysed by different mass spectrometry techniques (MALDI-TOF or ESI-MS). Alternatively, if the complexity of the peptide mixture does not allow the direct analysis by mass spectrometry, it is possible to separate the peptides using conventional techniques. Suitable separation techniques, which allow the separation of a complex peptide sample into multiple fractions, are known to the skilled person and include, but are not limited to isoelectric focusing, anion or cation exchange chromatography, reversed-phase HPLC, ion pair reversed-phase chromatography, affinity chromatography and the like. Though suitable in principle, techniques such as SDS PAGE, 2-dimensional gel electrophoresis, size-exclusion chromatography are less appropriate for the separation of peptides of generally limited length.

Several technologies to separate peptide digests by liquid chromatography have been described, including reversed-phase (RP)-HPLC, and 2-dimensional liquid chromatography. For peptide samples obtained from proteolytic digestions, 2D-LC approaches are particularly suitable for separation, providing also significant advantages with regard to automation and throughput. Also capillary electrophoresis (CE) is a method suitable for the separation of peptides. 2D-LC generally uses ion-exchange columns (usually, strong cation exchange, SCX) on-line coupled with a reversed phase column, operated in a series of cycles. In each cycle the salt concentration is increased in the ion-exchange column, in order to elute peptides according to their ionic charge into the reversed phase system. Herein, the peptides are separated on hydrophobicity by e.g. a gradient with CH₃CN.

In another aspect, the invention relates to a kit useful in the implementation of the methodology described herein. Thus, said kit comprises a set of reagents for detecting the expression levels of all the proteins, or genes coding said proteins according to the invention wherein said set consists of reagents for detecting antithrombin III, neutrophil cytosolic factor 2, glutathione peroxidase 3, acid haptoglobin alpha 2, haptoglobin beta, peroxiredoxin-2, complement factor I, basic haptoglobin alpha 1, and hemoglobin gamma proteins or the genes coding said proteins and, optionally, a reagent for detecting a housekeeping protein or the gene coding for said housekeeping protein.

In a particular embodiment, the set of reagents further consists of a reagent for detecting apolipoprotein A1 protein or the gene coding said protein. In another particular embodiment, the set of reagents further consists of a reagent for detecting fibrinogen gamma protein or the gene coding said protein.

In another particular embodiment, the reagents for detecting the proteins according to the invention, or the genes coding said proteins, comprise
(i) a set of nucleic acids capable of specifically hybridized with the genes coding said proteins, or
(ii) a set of antibodies, or a fragment thereof, capable of detecting an antigen, capable of specifically binding to said proteins.

Nucleic acids capable of specifically hybridized with the genes coding said proteins are, for example, one or more pairs of primer oligonucleotides for the specific amplification of fragments of the mRNAs (or of their corresponding cDNAs) of genes coding said proteins and/or one or more probes for the identification of one or more genes selected from said genes.

Antibodies, or a fragment thereof, capable of detecting an antigen, capable of specifically binding to the proteins according to the invention are, for example, monoclonal and polyclonal antibodies, antibody fragments, Fv, Fab, Fab' y F(ab')2, ScFv, diabodies, triabodies, tetrabodies and humanised antibodies.

Said reagents, specifically, the probes and the antibodies, may be fixed onto a solid support, such as a membrane, a plastic or a glass, optionally treated in order to facilitate fixation of said probes or antibodies onto the support. Said solid support, which comprises, at least, a set of antibodies capable of specifically binding to the protein of the present invention, or variant thereof, or probes specifically hybridized with the genes coding said proteins, respectively, may be used for the detection of the expression levels of the proteins according to the invention, or variant thereof, or of the genes coding said proteins, by means of array technology.

The kits of the invention optionally comprise additional reagents for detecting a polypeptide encoded by a housekeeping gene or the mRNA encoded by said housekeeping genes. The availability of said additional reagent allows the normalization of measurements taken in different samples (e.g. the test sample and the control sample) to exclude that the differences in expression of the different biomarkers are due to a different amount of total protein in the sample rather than to real differences in relative expression levels. Housekeeping genes, as used herein, relates to genes which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin.

The following example is provided as merely illustrative and is not to be construed as limiting the scope of the invention.

### EXAMPLE 1

### Detection of NASH/steatosis biomarkers in serum

### Samples

61 human serum samples ere obtained from different health centers:
   Control (15 samples)
   steatosis (15samples)
   NASH (22 samples)
   Hepatitis C (16samples)
   Hepatitis B (3 samples)
   Primary Biliar Cirrhosis (3 samples)
   Autoimmune hepatitis (2 samples)
   Wilson syndrome (1 sample)

Samples were classified into 8 different categories: Controls **(C)** for samples from healthy donors, steatosis **(S)** subjects, **NASH (N)** subjects, Hepatitis **C (VHC),** Hepatitis **C (VHC)** and autoimmune hepatitis **(AH)** as well as primary biliar cirrhosis **(CI)** and wilson syndrome **(W).**

### HSA depletion

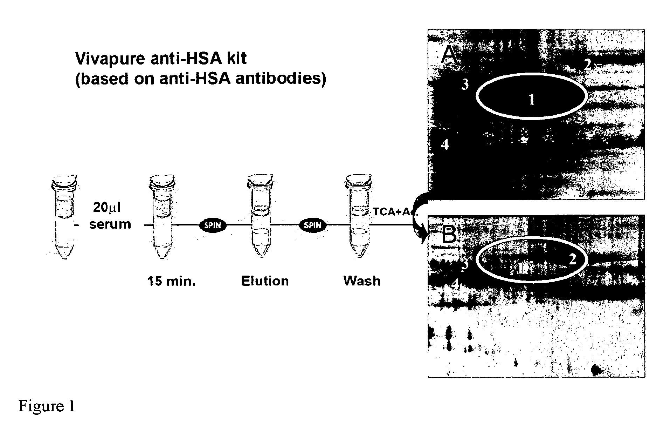

In order to deplete HSA, thus improving the 2D-electrophoresis separation, a commercial affinity resin-based kit (Vivapure anti-HSA kit, from Sartorius, Göttingen Germany) which contains fragments of antibodies raised against the human albumin was used. A small volume (20 µl) of serum was required to obtain approximately 300 µg of HSA-free serum proteins with this procedure. Figure 1 shows a schematic picture of the depletion process and 2D gel images showing the effect of HSA removal on resolution and sensitivity.

### Experimental design

HSA-depleted serum samples were diluted in a buffer containing 9M Urea, 2M thiourea and 65 mM DTT as well as biolytes (Bio-Rad) to denature and reduce the proteins. Samples in urea were loaded on Readystrips (Bio-Rad) ranging from pI 5 to pI 8 (first dimension, Isoelectrofocussing or Isoelectric point-depending separation). Isoelectrofocussing was carried out using the following voltage gradient:
S1 300V 0,01H
S2 300V 3H (linear)
S3 600V 6H (linear)
S4 600V 3H
S5 2000V 7H (linear)
S6 2000V 3H
S7 3500V 7H (linear)
S8 3500V HOLD

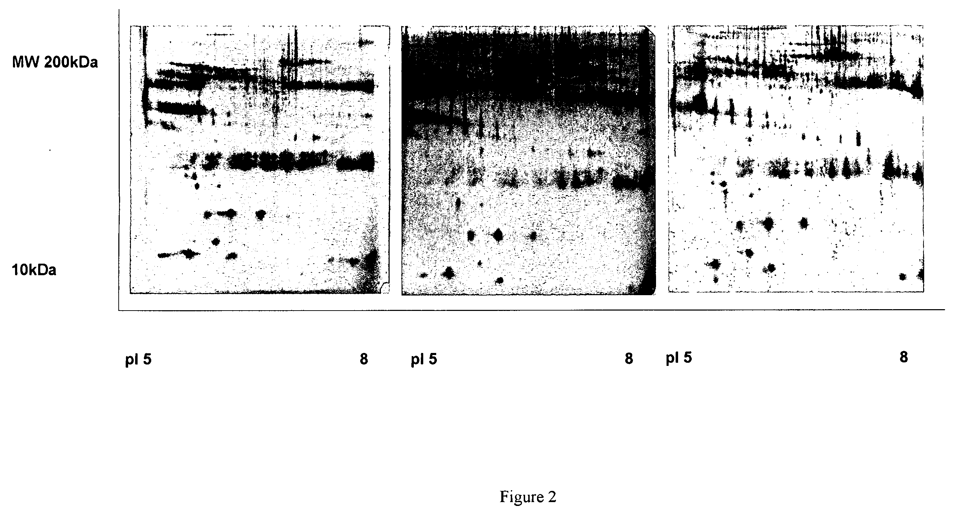

After equilibrating the strips sequentially in buffer containing Dithiothreitol (reduction) and Iodoacetamide (alkylation), they were placed on the top of a 18 x 20 centimetres, 12.5% polyacrylamide gel for the second dimension or SDS-PAGE (size-depending separation). Gels were run overnight at 80-85 V and after stopping the electrophoresis, they were fixed (10% Ethanol, 7 % acetic acid) and stained overnight using SYPRO RUBY fluorescent stain. Gel images were collected in a Typhoon TRIO UV-visible laser scanner with a suitable resolution and intensity for ulterior image analysis. Figure 2 shows some examples of 2D gel images.

Out of more than 100 2D gel images, the following sets were selected for image analysis:

| **Sample from subject** | **N° images** |
|---|---|
| Control | 13 images |
| steatosis | 13 images |
| NASH | 13 images |
| Hepatitis C | 8 images |
| Hepatitis B | 2 images |
| primary biliar cirrhosis | 3 images |
| autoimmune hepatitis | 2 images |
| Wilson syndrome | 1 image |

Once the analysis was completed using PDQuest software (Bio-Rad), 465 spots were matched for all members of the selected gel images and volume (intensity x height) values for each spot in each group was averaged. Statistical analysis of data allowed identifying the most significantly varying spots in each group and the ratio of the volume variation.

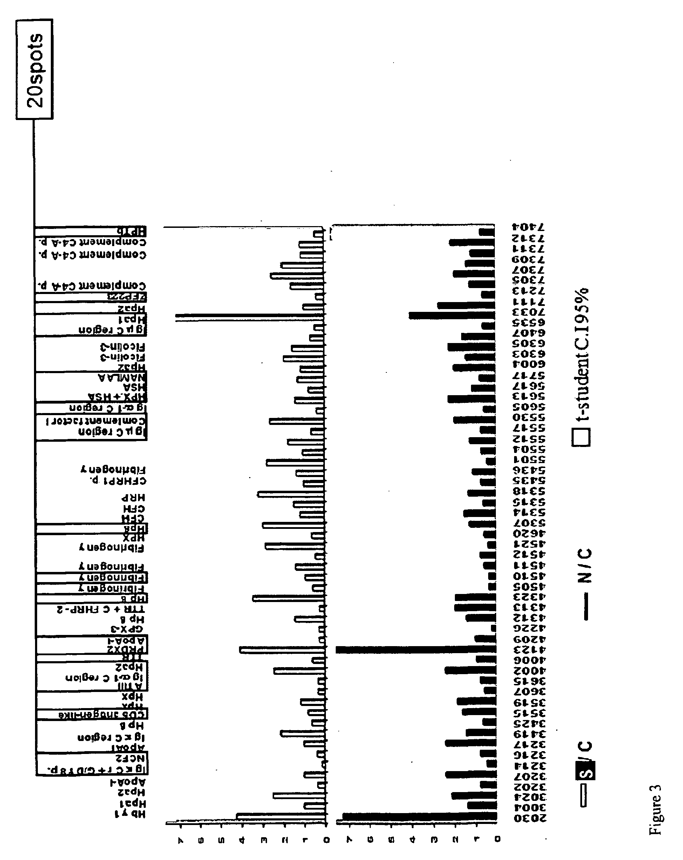

A preliminary list of 57 spots that varied in steatosis and/or in NASH samples with respect to controls above 1.5 fold (0.67>R>1.5) was obtained. Some of the spots were additionally significant according to the Student's t-test with a confidence interval of 95% (p<0.05). The 57 spots using a QtoFPremier mass spectrometer (MALDI-TOF) instrument (Waters) were identified and the list of down or up-regulated proteins for steatosis and/or NASH was inferred. Figure 3 shows the 57 selected spots with the volume values of steatosis and NASH spots relative to control spots (ratio) and the statistically significant spots (confidence interval >95%, 20 spots inside boxes). Table 2 shows the ratios of all the analysed diseases as well as the names of identified proteins for each spot (statistically significant, Student-t test C.I.>95%, names of proteins showing differential expression are shown in bold cursive).

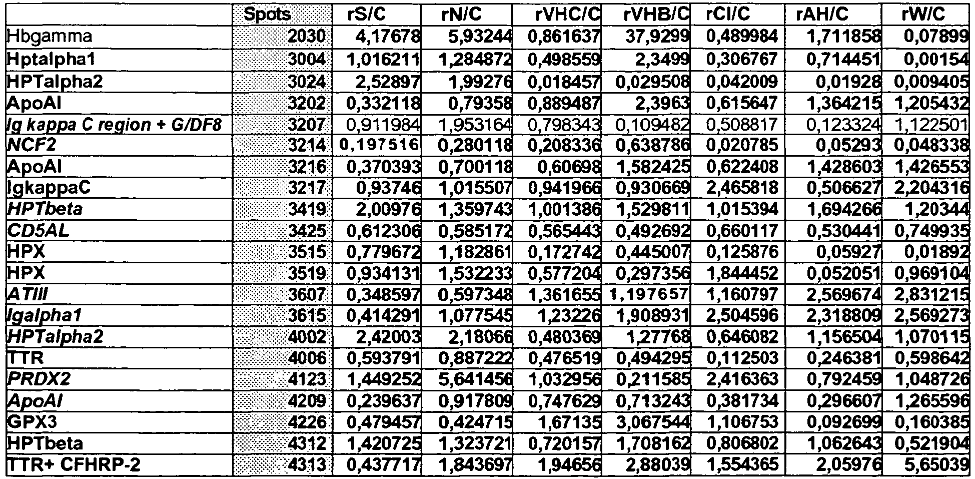

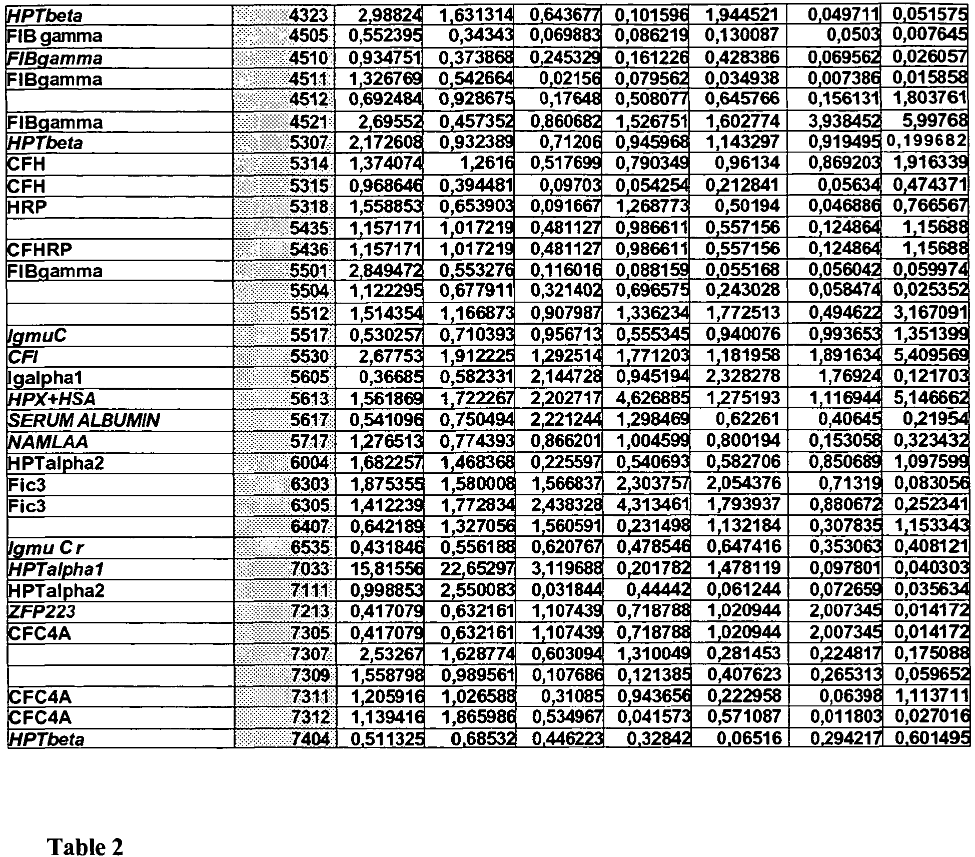

In a second step, the volume values of varying spots in steatosis and NASH gels were compared with the values for the same spots in the rest of the groups analysed by 2D electrophoresis (Hepatitis C and B, primary biliary cirrhosis, autoimmune hepatitis and Wilson syndrome). The spots showing differences in intensity with respect to the rest of the groups were choosen for a next round of selection. 11 spots were detected showing a quantitatively different trend in steatosis and/or NASH when compared to the rest of the groups. The 11 spots and their changes in expression (down-regulated N and down-regulated S means that protein is down-regulated only in NASH or only in steatosis when compared with control) are indicated in Table 3.

**Table 3: Proteins showing differential expression in steatosis vs. control samples and in NASH vs. control samples (left column) with indication of their relative change in expression (up- or down-regulated) (right column)**

| **Differential Control/steatosis and Control/NASH** | |
|---|---|
| Antithrombin III | Down-regulated |
| Neutrophil cytosilic factor 2 | Down-regulated |
| Glutathione peroxidase 3 | Up-regulated |
| Apolipoprotein AI | Down-regulated S |
| Acid haptoglobin alpha 2 | Up-regulated |
| Haptoglobin beta | Up-regulated |
| Peroxiredoxin-2 | Up-regulated |
| Fibrinogen gamma | Down-regulated N |
| Complement factor I | Up-regulated |
| Basic haptoglobin alpha 1 | Up-regulated |
| Hemoglobin gamma | Up-regulated |

### Validation

The validation of the selected proteins as biomarkers of NASH was carried out using western blot technique, an affinity-based procedure that combines electrophoretic separation of proteins with specific detection by means of antibodies raised against specific proteins.

Affinity test allows deciding to use commercially available antibodies and to test their avidity and specificity as a previous step for the use of those antibodies in a more sensitive and quantitative technique named ELISA (Enzyme Linked ImmunoSorbent Assay).

## Claims

1. An *in vitro* method for discriminating between patients suffering NASH or steatosis and patients suffering from other types of liver diseases, comprising:
a) detecting and quantifying in a sample from said subject the levels of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said proteins and
b) comparing the results obtained in step a) with reference values of the levels of said proteins or of the genes encoding said proteins,
wherein
(i) if the levels of antithrombin III and neutrophil cytosolic factor 2 proteins or of the genes encoding said proteins are lower than reference values for each of said protein, or gene and
(ii) if the levels of glutathione peroxidase 3, acid haptoglobin alpha 2, haptoglobin beta, peroxiredoxin-2, complement factor I, basic haptoglobin alpha 1 and hemoglobin gamma proteins or of the genes encoding said proteins are higher than reference values for each of said proteins or genes,
then the subject suffers from NASH or steatosis.

2. Method according to claim 1 further comprising detecting and quantifying in step (i) the levels of apolipoprotein AI or of the gene encoding said protein, wherein if said levels are lower than reference values, then the subject suffers from, or is predisposed to develop steatosis.

3. Method according to claim 1 further comprising detecting and quantifying in step (i) the levels of fibrinogen gamma or of a gene encoding said protein wherein if said levels are lower than reference values, then the subject suffers from, or is predisposed to develop NASH.

4. An *in vitro* method for monitoring the effect of the therapy administered to a subject suffering NASH or steatosis, comprising:
a) detecting and quantifying the levels of antithrombin III, neutrophil cytosolic factor 2, glutathione peroxidase 3, acid haptoglobin alpha 2, haptoglobin beta, peroxiredoxin-2, complement factor I, basic haptoglobin alpha 1 and hemoglobin gamma or of the genes encoding said proteins and
b) comparing the results obtained in step (a) with reference values for the levels of said proteins or genes encoding said proteins,
wherein
(i) if the levels of antithrombin III and neutrophil cytosolic factor 2 proteins or of the genes encoding said proteins are higher than reference values and
(ii) if the levels of glutathione peroxidase 3, acid haptoglobin alpha 2, haptoglobin beta, peroxiredoxin-2, complement factor I, basic haptoglobin alpha 1 and hemoglobin gamma proteins or of the genes encoding said proteins are lower than reference values then
the therapy administered to said subject has been effective or is being effective.

5. Method according to claim 4, further comprising detecting the levels of apolipoprotein A1 protein or of the gene encoding said protein wherein if said levels are lower than a reference value, then the therapy administered to said subject has not been effective or is not being effective for the treatment of steatosis.

6. Method according to claim 4 further comprising detecting the levels of fibrinogen gamma or of the gene encoding said protein wherein if said levels are lower than a reference value, then the therapy administered to said subject has not been effective or is not being effective for the treatment of NASH.

7. Method according to anyone of claims 1 to 6, wherein the sample is serum, plasma or HSA-depleted serum or plasma.

8. Method according to anyone of claims 1 to 7, wherein the levels of the proteins are determined by western blot, ELISA or two-dimensional electrophoresis and staining.

9. A kit comprising a set of reagents, wherein said set consists of reagents for detecting antithrombin III, neutrophil cytosolic factor 2, glutathione peroxidase 3, acid haptoglobin alpha 2, haptoglobin beta, peroxiredoxin-2, complement factor I, basic haptoglobin alpha 1, and hemoglobin gamma proteins or the genes coding said proteins and, optionally, a reagent for detecting a housekeeping protein or the gene coding for said housekeeping protein.

10. Kit according to claim 9 further comprising a reagent for detecting apolipoprotein A1 protein or for detecting the gene coding said protein.

11. Kit according to claim 9 further comprising a reagent for detecting fibrinogen gamma protein or the gene coding said protein.

12. Kit according to anyone of claims 9 to 11, wherein the set of reagents is selected from the group of:
a) A set of nucleic acids capable of specifically hybridising with the genes coding said proteins and
b) A set of antibodies, or fragments thereof capable of specifically binding to said proteins.

## Patentansprüche

1. In-vitro-Verfahren zur Unterscheidung von Patienten, die an NASH oder Steatose leiden, und Patienten, die an anderen Arten von Lebererkrankungen leiden, umfassend:
a) den Nachweis und die Quantifizierung der Spiegel an Antithrombin III, neutrophilem cytosolischem Faktor 2, Glutathionperoxidase 3, saurem Haptoglobin-alpha 2, Haptoglobin-beta, Peroxiredoxin-2, Komplementfaktor I, basischem Haptoglobin-alpha 1 und Hämoglobin-gamma oder der Gene, die diese Proteine codieren, in einer Probe von dem Individuum, und
b) den Vergleich der in Schritt a) erhaltenen Werte mit Bezugswerten der Spiegel der Proteine oder der Gene, die diese Proteine codieren;
wobei,
(i) wenn die Spiegel der Proteine Antithrombin III und neutrophiler cytosolischer Faktor 2 oder der Gene, die diese Proteine codieren, niedriger sind als die Bezugswerte für jedes dieser Proteine oder Gene, und
(ii) wenn die Spiegel der Proteine Glutathionperoxidase 3, saures Haptoglobin-alpha 2, Haptoglobin-beta, Peroxiredoxin-2, Komplementfaktor I, basisches Haptoglobin-alpha 1 und Hämoglobin-gamma oder der Gene, die diese Proteine codieren, höher sind als die Bezugswerte für jedes dieser Proteine oder Gene,
das Individuum dann an NASH oder Steatose leidet.

2. Verfahren gemäß Anspruch 1, ferner umfassend den Nachweis und die Quantifizierung der Spiegel an Apolipoprotein AI oder des Gens, das das Protein codiert, in Schritt (i), wobei, wenn die Spiegel niedriger sind als die Bezugswerte, das Individuum dann an einer Steatose leidet oder empfänglich dafür ist, eine Steatose zu entwickeln.

3. Verfahren gemäß Anspruch 1, ferner umfassend den Nachweis und die Quantifizierung der Spiegel an Fibrinogen-gamma oder eines Gens, das das Protein codiert, in Schritt (i), wobei, wenn die Spiegel niedriger sind als die Bezugswerte, das Individuum dann an NASH leidet oder empfänglich dafür ist, eine NASH zu entwickeln.

4. In-vitro-Verfahren zur Überwachung der Wirkung einer an ein Individuum, welches an NASH oder Steatose leidet, verabreichten Therapie, umfassend:
a) den Nachweis und die Quantifizierug der Spiegel an Antithrombin III, neutrophilem cytosolischem Faktor 2, Glutathionperoxidase 3, saurem Haptoglobin-alpha 2, Haptoglobin-beta, Peroxiredoxin-2, Komplementfaktor I, basischem Haptoglobin-alpha 1 und Hämoglobin-gamma oder der Gene, die diese Proteine codieren, und
b) den Vergleich der in Schritt a) erhaltenen Werte mit Bezugswerten für die Spiegel der Proteine oder der Gene, die diese Proteine codieren;
wobei
(i) wenn die Spiegel der Proteine Antithrombin III und neutrophiler cytosolischer Faktor 2 oder der Gene, die diese Proteine codieren, höher sind als die Bezugswerte, und
(ii) wenn die Spiegel der Proteine Glutathionperoxidase 3, saures Haptoglobin-alpha 2, Haptoglobin-beta, Peroxiredoxin-2, Komplementfaktor I, basisches Haptoglobin-alpha 1 und Hämoglobin-gamma oder der Gene, die diese Proteine codieren, niedriger sind als die Bezugswerte,
die an das Individuum verabreichte Therapie dann wirksam war oder wirksam ist.

5. Verfahren gemäß Anspruch 4, ferner umfassend den Nachweis der Spiegel an Apolipoprotein AI oder des Gens, das das Protein codiert, wobei, wenn die Werte niedriger sind als ein Bezugswert, dann die an das Individuum verabreichte Therapie zur Behandlung von Steatosis nicht wirksam gewesen ist oder nicht wirksam ist.

6. Verfahren gemäß Anspruch 4, ferner umfassend den Nachweis der Spiegel an Fibrinogen-gamma oder des Gens, das das Protein codiert, wobei, wenn die Spiegel niedriger sind als ein Bezugswert, die an das Individuum verabreichte Therapie zur Behandlung von NASH nicht wirksam gewesen ist oder nicht wirksam ist.

7. Verfahren gemäß einem der Ansprüche 1 bis 6, wobei die Probe Serum, Plasma oder HSA-verarmtes Serum oder Plasma ist.

8. Verfahren gemäß einem der Ansprüche 1 bis 7, wobei die Proteinspiegel durch Western-Blot, ELISA oder zweidimensionale Elektrophorese und Färbung bestimmt werden.

9. Kit umfassend einen Satz an Reagenzien, wobei der Satz aus Reagenzien zum Nachweis von Antithrombin III, neutrophilem cytosolischem Faktor 2, Glutathionperoxidase 3, saurem Haptoglobin-alpha 2, Haptoglobin-beta, Peroxiredoxin-2, Komplementfaktor I, basischem Haptoglobin-alpha 1 und Hämoglobin-gamma oder von Genen, die diese Protein codieren, und, gegebenenfalls, einem Reagenz zum Nachweis eines Housekeeping-Proteins oder dem Gen, das das Housekeeping-Protein codiert, besteht.

10. Kit gemäß Anspruch 9, ferner umfassend ein Reagenz zum Nachweis des Apolipoprotein AI-Proteins oder zum Nachweis des Gens, das das Protein codiert.

11. Kit gemäß Anspruch 9, ferner umfassend ein Reagenz zum Nachweis des Fibrinogen-gamma-Proteins oder des Gens, das das Protein codiert.

12. Kit gemäß einem der Ansprüche 9 bis 11, wobei der Reagenziensatz ausgewählt ist aus der Gruppe bestehend aus:
a) einem Satz an Nucleinsäuren, welche fähig sind, mit den Genen, die diese Proteine codieren, spezifisch zu hybridisieren, und
b) einem Satz an Antikörpern oder Fragmenten davon, die fähig sind, an die Proteine zu binden.

## Revendications

1. Procédé *in vitro* pour faire la différence entre les patients souffrant de stéatohépatite non alcoolique (NASH) ou de stéatose et les patients souffrant d'autres types de maladies hépatiques, comprenant :
a) la détection et la quantification dans un échantillon provenant d'un tel sujet des taux d'antithrombine III, de facteur cytosolique neutrophile 2, de glutathion-peroxydase 3, d'haptoglobine acide alpha 2, d'haptoglobine bêta, de peroxirédoxine 2, de facteur du complément I, d'haptoglobine basique alpha 1 et d'hémoglobine gamma ou des gènes codant pour lesdites protéines, et
b) la comparaison des résultats obtenus dans l'étape a) avec des valeurs de référence des taux desdites protéines ou des gènes codant pour lesdites protéines,
dans lequel
(i) si les taux de protéines consistant en antithrombine III et facteur cytosolique neutrophile 2 ou des gènes codant pour lesdites protéines sont inférieurs aux valeurs de référence pour chacune desdites protéines, ou desdits gènes, et
(ii) si les taux de protéines consistant en glutathion-peroxydase 3, haptoglobine acide alpha 2, haptoglobine bêta, peroxirédoxine 2, facteur du complément I, haptoglobine basique alpha et hémoglobine gamma ou des gènes codant pour lesdites protéines sont supérieurs aux valeurs de référence pour chacune desdites protéines, ou desdits gènes,
alors le sujet souffre de NASH ou de stéatose.

2. Procédé suivant la revendication 1, comprenant en outre la détection et la quantification dans l'étape (i) des taux d'apolipoprotéine AI ou du gène codant pour ladite protéine, dans lequel, si lesdits taux sont inférieurs aux valeurs de référence, alors le sujet souffre de, ou est prédisposé à développer une, stéatose.

3. Procédé suivant la revendication 1, comprenant en outre la détection et la quantification dans l'étape (i) des taux de fibrinogène gamma ou d'un gène codant pour ladite protéine, dans lequel, si lesdits taux sont inférieurs aux valeurs de référence, alors le sujet souffre de, ou est prédisposé à développer une, NASH.

4. Procédé *in vitro* pour contrôler l'effet de la thérapie administrée à un sujet souffrant de NASH ou de stéatose, comprenant :
a) la détection et la quantification des taux d'antithrombine III, de facteur cytosolique neutrophile 2, de glutathion-peroxydase 3, d'haptoglobine acide alpha 2, d'haptoglobine bêta, de peroxirédoxine 2, de facteur du complément I, d'haptoglobine basique alpha 1 et d'hémoglobine gamma ou des gènes codant pour lesdites protéines, et
b) la comparaison des résultats obtenus dans l'étape (a) avec des valeurs de référence des taux desdites protéines ou desdits gènes codant pour lesdites protéines,
dans lequel
(i) si les taux de protéines consistant en antithrombine III et facteur cytosolique neutrophile 2 ou des gènes codant pour lesdites protéines sont supérieurs aux valeurs de référence, et
(ii) si les taux de protéines consistant en glutathion-peroxydase 3, haptoglobine acide alpha 2, haptoglobine bêta, peroxirédoxine 2, facteur du complément I, haptoglobine basique alpha et hémoglobine gamma ou des gènes codant pour lesdites protéines sont inférieurs aux valeurs de référence, alors
la thérapie administrée audit sujet a été efficace ou est efficace.

5. Procédé suivant la revendication 4, comprenant en outre la détection des taux de protéine apolipoprotéine A1 ou du gène codant pour ladite protéine, dans lequel, si lesdits taux sont inférieurs à une valeur de référence, alors la thérapie administrée audit sujet n'a pas été efficace ou n'est pas efficace pour le traitement de la stéatose.

6. Procédé suivant la revendication 4, comprenant en outre la détection des taux de fibrinogène gamma ou du gène codant pour ladite protéine, dans lequel, si lesdits taux sont inférieurs à une valeur de référence, alors la thérapie administrée audit sujet n'a pas été efficace ou n'est pas efficace pour le traitement de la NASH.

7. Procédé suivant l'une quelconque des revendications 1 à 6, dans lequel l'échantillon est un échantillon de sérum, de plasma ou bien de sérum ou plasma présentant une déplétion de HSA.

8. Procédé suivant l'une quelconque des revendications 1 à 7, dans lequel les taux des protéines sont déterminés par transfert d'empreinte par la méthode Western, analyse ELISA ou électrophorèse bidimensionnelle et coloration.

9. Kit comprenant une série de réactifs, dans lequel ladite série consiste en réactifs pour détecter les protéines consistant en antithrombine III, facteur cytosolique neutrophile 2, glutathion-peroxydase 3, haptoglobine acide alpha 2, haptoglobine bêta, peroxirédoxine 2, facteur du complément I, haptoglobine basique alpha 1 et hémoglobine gamma ou les gènes codant pour lesdites protéines, et, facultativement, un réactif pour détecter une protéine d'entretien ou le gène codant pour ladite protéine d'entretien.

10. Kit suivant la revendication 9, comprenant en outre un réactif pour détecter la protéine apolipoprotéine A1 ou pour détecter le gène codant pour ladite protéine.

11. Kit suivant la revendication 9, comprenant en outre un réactif pour détecter la protéine fibrinogène gamma ou le gène codant pour ladite protéine.

12. Kit suivant l'une quelconque des revendications 9 à 11, dans lequel la série de réactifs est choisie dans le groupe consistant en:
a) une série d'acides nucléiques capables de s'hybrider spécifiquement avec les gènes codant pour lesdites protéines, et
b) une série d'anticorps, ou de leurs fragments, capables de se lier spécifiquement auxdites protéines.
